# EUROPÄISCHE PATENTSCHRIFT

(11) **EP 3 746 004 B1**
(45) Veröffentlichungstag und Bekanntmachung des Hinweises auf die Patenterteilung: **08.11.2023**
(21) Anmeldenummer: 19703964.7
(22) Anmeldetag: 30.01.2019
(51) Int. Cl.: A61F 2/44, A61F 2/30, A61F 2/46

(54) **CAGEPOSITIONIERSYSTEM SOWIE CAGE UND POSITIONIERINSTRUMENT FÜR EIN SOLCHES CAGEPOSITIONIERSYSTEM**
CAGE POSITIONING SYSTEM AND CAGE AND POSITIONING INSTRUMENT FOR A CAGE POSITIONING SYSTEM OF THIS KIND
SYSTÈME DE POSITIONNEMENT DE CAGE AINSI QUE CAGE ET INSTRUMENT DE POSITIONNEMENT POUR UN TEL SYSTÈME DE POSITIONNEMENT DE CAGE

(30) Priorität: 30.01.2018 DE 102018201399
(43) Veröffentlichungstag der Anmeldung: 09.12.2020
(73) Patentinhaber: Premiere Medical GmbH, 89155 Erbach (DE)
(72) Erfinder: VAZIFEHDAN, Farzam, 70195 Stuttgart (DE); NILSSON, C. Michael, Moreland Hills, Ohio 44022 (US); SCHÖNHÖFFER, Helmut, 89155 Erbach (DE)
(74) Vertreter: Kohler Schmid Möbus Patentanwälte
(86) Internationale Anmeldenummer: PCT/EP2019/052241
(87) Internationale Veröffentlichungsnummer: WO 2019/149749

(56) Entgegenhaltungen:
- US-A1- 2004 153 065
- US-A1- 2006 235 426
- US-A1- 2008 109 005
- US-A1- 2008 140 085

## Beschreibung

Die Erfindung betrifft ein Cagepositioniersystem sowie einen Cage und ein Positionierinstrument für ein solches Cagepositioniersystem.

Bei traumatischen und/oder degenerativen Erkrankungen der lumbalen Wirbelsäule wird zur Korrektur und Stabilisierung der Wirbelsäule häufig eine intervertebrale Fusion von Wirbelkörpern durchgeführt. Hierzu wird die geschädigte Bandscheibe entfernt und mittels eines in deren Bandscheibenfach zu implantierenden Bandscheibenplatzhalters, dem sogenannten Cage, ersetzt. Die an das Bandscheibenfach angrenzenden Wirbel werden durch einen Fixateur interne im Wege einer transpedikulären Instrumentierung der Wirbelsäule relativ zueinander stabilisiert. Der Stabilisierungseffekt kann gegebenenfalls durch eine von vorne auf die Wirbelkörper geschraubte Metallplatte, eine sogenannte ventrale Plattenosteosynthese, noch weiter gesichert werden. Bei derlei Versteifungsoperationen werden in Abhängigkeit vom jeweiligen operativen Zugangsweg die sogenannte "PLIF" (posterior lumbar intervertebral fusion), "ALIF" (anterior lumbar intervertebral fusion) und "TLIF' ("transforaminal lumbar interbody fusion") unterschieden.

Der zumeist aus Titan bestehende implantierte Cage verwächst in der Regel innerhalb von sechs bis zwölf Wochen fest mit den angrenzenden Wirbelkörpern. Vor der eigentlichen Operation wird üblicherweise ein jeweilig notwendiges individuelles Korrekturausmaß, d. h., die jeweilige Höhe und der Winkel des Bandscheibenfaches und damit die Stellung der an das Bandscheibenfach begrenzenden Wirbel, anhand bildgebender Verfahren bestimmt. Sobald der operierte Wirbelsäulenabschnitt in einer bestimmten Lage stabil ist, werden zwangsläufig die anderen Wirbelsäulenbereiche anders bewegt und belastet, was im Laufe der Zeit zu Verschleißerscheinungen führen kann. Dies ist insbesondere dann der Fall, wenn der implantierte Cage die Wirbel in einer suboptimalen Position fixiert bzw. selbst fehlerhaft positioniert wurde.

Die heutigen Operationsverfahren sind aufgrund nicht zu vermeidender präoperativer Mess- und Berechnungsfehler sowie insbesondere auch intraoperativer Schwierigkeiten, den Cage in der individuell richtigen Lage und Position im Bandscheibenfach zu positionieren, durchaus risikobehaftet. Die am Markt verfügbaren Cage-Implantate müssen darüber hinaus in einer ausreichenden Zahl von Varianten vorgehalten werden, um individuellen Anforderungen an die Winkel- und Höheneinstellung des Bandscheibenfaches auch nur ansatzweise Genüge zu tun.

Es ist deshalb die Aufgabe der vorliegenden Erfindung, ein Cagepositioniersystem bereitzustellen, mit dem ein zu implantierende Cage auf vereinfachte, sicherere, weniger traumatische und kontrolliertere Weise in der individuell richtigen Lage und Position im vorgegebenen Bandscheibenfach positioniert werden kann. Das Cage-Positioniersystem soll dabei nach Möglichkeit patientenseitigen individuellen anatomischen Anforderungen universeller gerecht werden.

Die das Cagepositioniersystem betreffende Aufgabe wird durch ein Cagepositioniersystem mit den in Anspruch 1 angegebenen Merkmalen gelöst. Der erfindungsgemäße Cage ist in Anspruch 17 angegeben und das Positionierinstrument weist die in Anspruch 18 angegebenen Merkmale auf.

Das chirurgische Cagepositioniersystem dient dem implantologischen Ersatz einer Bandscheibe, bevorzugt im Bereich der lumbalen Wirbelsäule des Menschen. Das Cagepositioniersystem umfasst ein Positionierinstrument mit einem Betätigungssegment und mit einem Instrumentiersegment, der sich vom Betätigungssegment in Richtung der Längsachse des Positionierinstruments wegerstreckt. Das Instrumentiersegment weist einen ersten Kopplungszinken und einen ersten Manipulatorzinken auf, die an ihrem freien Endabschnitt jeweils mit einem Koppelglied versehen sind. Der Manipulatorzinken ist mittels einer, bevorzugt am Betätigungssegment des Positionierinstruments angeordneten, Handhabe relativ zum Kopplungszinken in axialer Richtung (hin- und her) verschiebbar angeordnet. Ein solches Positionierinstrument ist aus dem Patentdokument US2006/235436 A1 bekannt.

Das Cagepositioniersystem umfasst ferner einen als Bandscheibenersatz zu implantierenden Cage mit einer Einstecköffnung für das Instrumentiersegment und mit Kopplungsmitteln, über die die Koppelglieder des Instrumentiersegments jeweils drehgelenkig mit dem Cage verkoppelbar sind. die Dopplungsmittel und die Koppelglieder des am Cage angekoppelten Positionierinstruments wirken derart zusammen, dass der Cage mittels des Manipulatorzinkens um eine durch das Koppelglied des Kopplungszinkens und das Kopplungsmittel des Cages definierte erste Drehachse relativ zur Längsachse des Positionierinstruments verschwenkbar ist.

Durch das erfindungsgemäße Cagepositioniersystem kann der Cage in Richtung seiner Transversalachse, entlang derer der Cage in der Regel seine größte Erstreckung aufweist, in den Operationssitus und in das zur Aufnahme des Cages vorgesehene Bandscheibenfach eingeführt und erst im Bandscheibenfach in seine vorgegebene Drehlage relativ zu den angrenzenden Wirbelkörpern überführt werden. Durch den reversiblen Anschluss des Manipulatorzinkens am Cage behält der Chirurg immer die vollständige Kontrolle über den jeweiligen Schwenkwinkel des Cages relativ zum Positionierinstrument. Dadurch kann der Cage insgesamt einfacher, zügiger, atraumatischer und exakter an einer vorgegebenen Position und in vorgegebener Lage im Bandscheibenfach positioniert und abgesetzt werden. Langfristig kann dadurch auch unerwünschten Verschleißerscheinungen und Fehlhaltungen der Wirbelsäule infolge des chirurgischen Eingriffs, insbesondere aufgrund einer suboptimalen Positionierung des Cages im Bandscheibenfach, entgegengewirkt werden.

Durch das dem Koppelglied des Manipulatorzinkens zugeordnete Kopplungsmittel des Cages ist nach der Erfindung beim Verschwenken des Cages eine translatorische Verstellung des Koppelglieds des Manipulatorzinkens entlang einer nichtlinearen Bewegungsbahn relativ zum Cage bewirkt. Dadurch kann der Cage im Bandscheibenfach über einen ausreichend großen Schwenkwinkel relativ zum Kopplungszinken bzw. der Längsachse des Positionierinstruments geschwenkt werden.

Das zum Koppelglied des Manipulatorzinkens korrespondierende Kopplungsmittel des Cages ist nach der Erfindung insbesondere als eine nichtlineare Vertiefung des Cages in Form einer nichtlinearen Nut oder eines nichtlinearen Langlochs ausgebildet. Beim Verschwenken fungiert das Langloch als eine Steuerkulisse für das andere jeweilig darin eingreifende Koppelglied des Manipulatorzinkens. Es versteht sich, dass die Einstecköffnung des Cages für einen jeweilig vorgegebenen maximalen Schwenkwinkel ausgelegt sein muss. Soll der Cage am Positionierinstrument mit seiner Transversalachse parallel oder im Wesentlichen parallel zur Längsachse des Positionierinstruments ausgerichtbar sein, so kann die Einstecköffnung vorteilhaft zur Seitenfläche des Cages hin offen ausgeführt sein. Der am Instrumentiersegment des Positionierinstruments angekoppelte Cage ist nach der Erfindung durch Betätigen des Manipulationszinkens, insbesondere aus aus seiner Neutralstellung (mit zur Längsachse parallel oder im Wesentlichen parallel verlaufend angeordneter Transversalachse), bevorzugt über einen Schwenkwinkel β mit 0° ≤ β ≤ 80° relativ zur Längsachse des Positionierwerkzeugs verschwenkbar (drehbar). Dadurch kann der Cage unabhängig vom gewählten operativen Zugangsweg auf einfache Weise in seine jeweilig vorgegebene Relativlage im Bandscheibenfach gedreht und an der vorgegebenen Position im Bandscheibenfach abgesetzt werden.

Das Koppelglied des ersten Manipulatorzinkens und/oder das Koppelglied des ersten Kopplungszinkens können nach der Erfindung jeweils als ein Profilvorsprung mit bevorzugt kreisrunder Querschnittsform ausgebildet sein. Der Profilvorsprung erstreckt sich von dem jeweiligen Zinken in einer radialen Richtung weg. Die Koppelglieder weisen bevorzugt eine kreiszylindrische Form auf. Dadurch können während des Implantierens ausreichend große Kräfte auf den Cage übertragen werden, um diesen etwa gegen einen Widerstand in seiner vorgegebene Position im Bandscheibenfach absetzten zu können. Die Kanten der Koppelglieder können dabei angefast oder gerundet ausgeführt sein. Dadurch kann einem unerwünschten Verkanten oder ungewollten Verhaken des Instrumentiersegments mit dem Cage entgegengewirkt werden.

Für eine verliersicheres mechanisches Verkoppeln des Kopplungszinkens mit dem Cage weist das Positionierinstrument vorzugsweise ein erstes Arretiermittel auf. Dieses Arretiermittel ist bevorzugt im oder am Grundkörper des Instrumentiersegments längsverschieblich gelagert und relativ zum Koppelzinken in axialer Richtung zwischen einer das Ablösen des mit dem Cage verkoppelten Kopplungszinken ermöglichenden Freigabestellung und einer den am Cage angekoppelten Kopplungszinken arretierenden Arretierstellung verschiebbar. Insgesamt kann dadurch eine nochmals laststabilere und zugleich lösbare Befestigung des Instrumentiersegments am Cage realisiert werden.

Die Einstecköffnung des Cages kann nach der Erfindung eine derartige lichte Höhe aufweisen, dass sich das Arretiermittel in seiner Arretierstellung in Richtung der Vertikalachse des Cages an einem die Einstecköffnung begrenzenden Wandbereich des Cages abstützen kann. Dadurch kann der Kopplungszinken des Positionierinstruments auf einfach Weise in seiner Ankopplungsposition am Cage gesichert werden.

Das erste Arretiermittel ist bevorzugt über das Betätigungssegment des Positionierinstruments betätigbar. So kann das Betätigungssegment beispielsweise eine rotierbar und/oder translatorisch verschiebbar gelagerte Handhabe aufweisen, die mit dem ersten Arretiermittel bewegungsgekoppelt ist. Diese Handhabe kann am Betätigungssegment reversibel anschließbar oder in das Betätigungssegment bei Bedarf einsteckbar sein.

Das Instrumentiersegment kann nach einer bevorzugten Ausführungsform der Erfindung einen zum ersten Kopplungszinken parallel verlaufend angeordneten zweiten Kopplungszinken aufweisen. Dadurch kann das Positionierinstrument nochmals sicherer am Cage reversibel angekoppelt werden. Das erste Arretiermittel ist bei dieser Ausführungsform in seiner Arretierstellung bevorzugt mit seinem freien Endabschnitt zwischen dem ersten und dem zweiten Kopplungszinken des Instrumentiersegments angeordnet, um so ein Approximieren der beiden Kopplungszinken relativ zueinander sowie ein damit einhergehendes Diskonnektieren der beiden Kopplungszinken vom Cage zu unterbinden. Durch das Überführen des Arretiermittels in seine Arretierposition zwischen den beiden Kopplungszinken können diese bei Bedarf relativ zueinander aufgespreizt werden. Diese Ausführungsform erlaubt ein besonders sicheres und nochmals zuverlässigeres Ankoppeln des Instrumentiersegments am Cage. Zugleich können dadurch Momente im Kupplungsbereich nochmals besser aufgenommen werden. Ein vorzeitiges Diskonnektieren des Cages vom Positionierinstrument kann dadurch besonders zuverlässig ausgeschlossen werden.

Das Positionierinstrument kann nach der Erfindung ein zweites Arretiermittel aufweisen, das im oder am Grundkörper des Instrumentiersegments längsverschieblich gelagert ist und welches relativ zum Manipulatorzinken (sowie auch zum Kopplungszinken) in axialer Richtung zwischen einer Freigabestellung und einer den Manipulatorzinken am Cage arretierenden Arretierstellung verschiebbar ist. Insgesamt können dadurch mittels des Manipulators größere Kräfte auf den Cage übertragen werden.

Weist die Einstecköffnung des Cages eine derartige lichte Höhe auf, dass sich das zweite Arretiermittel in seiner Arretierstellung in Richtung der Vertikalachse des Cages an einem die Einstecköffnung begrenzenden Wandbereich des Cages abstützen kann, so kann der Manipulatorzinken auf denkbar einfache Weise in seiner mit dem Cage verkoppelten Einsatzposition gesichert werden.

Das Instrumentiersegment kann erfindungsgemäß einen zum ersten Manipulatorzinken parallel verlaufenden zweiten Manipulatorzinken aufweisen. Der vorstehend erläuterte zweite Arretiermittel ist bei dieser Bauart in seiner Arretierstellung vorzugswiese abschnittsweise, insbesondere mit seinem freien Endabschnitt, zwischen den beiden Manipulatorzinken angeordnet, um ein Approximieren derselben (und damit deren Diskonnektieren vom Cage) zu sperren. Dadurch können beide Manipulatorzinken mit nur einem Arretiermittel in ihrer mit dem Cage verkoppelten Funktionsstellung gesichert werden.

Der erste und der zweite Manipulatorzinken sind in Richtung der Längsachse des Positionierinstruments vorzugsweise synchronisiert verschiebbar am Instrumentiersegment angeordnet. Die beiden Manipulatorzinken können dazu beispielsweise aneinander befestigt oder einstückig miteinander ausgebildet sein. Sind die Koppelglieder der beiden Manipulatorzinken jeweils in Form eines Profilvorsprungs ausgebildet, so weisen die Koppelglieder vom jeweiligen Manipulatorzinken vorzugsweise in einander entgegengesetzte Richtungen weg.

Das vorgenannte Arretiermittel kann in seiner Arretierstellung zwischen dem einen Manipulatorzinken und dem Cage oder zwischen den beiden Manipulatorzinken bevorzugt reibschlüssig gehalten. Dadurch kann das Arretiermittel gemeinsam mit dem Manipulatorzinken synchron mitbewegt werden, ohne aus seiner Arretierstellung herausbewegt zu werden

Nach einer bevorzugten Ausführungsform der Erfindung ist der einzelne Manipulatorzinken bzw. sind die beiden vorgenannten Manipulatorzinken über ein Betätigungssegment betätigbar, d.h. in einer zur Längsachse des Positionierinstruments axialen Richtung verschiebbar.

Das erste Arretiermittel und/oder das vorgenannte zweite Arretiermittel ist/sind nach einer bevorzugten Ausführungsform der Erfindung in Form eines stabförmigen Arretierschiebers ausgeführt. Der Arretierschieber kann dabei aus Kunststoff oder aus Metall bestehen.

Der Arretierschieber ist vorzugsweise in einem Führungskanal des Instrumentiersegments geführt.

Der bzw. die Manipulatorzinken sind vorzugsweise an einem Manipulator(schieber) angeordnet, insbesondere angeformt. Der Manipulator kann als eine Hülse ausgebildet sein. Der vorgenannte zweite Arretierschieber kann innerhalb der Hülse geführt sein.

Nach der Erfindung ist/sind das erste und/oder das zweite Arretiermittel vorzugsweise über das Betätigungssegment betätigbar. Dies vereinfacht die Handhabung und erlaubt ein wenig traumatisches Implantieren des Cages.

Unter fertigungstechnischen Gesichtspunkten sowie auch unter Kostengesichtspunkten sind das Instrumentiersegment und das Betätigungssegment bevorzugt jeweils als ein Instrumentenmodul ausgeführt, die miteinander lösbar verkoppelbar sind. Durch einen solchen modularen Aufbau kann zudem die Einsatzbreite des Cage-Positioniersystems vergrößert werden. So kann das Cagepositioniersystem mehrere Instrumentiersegmente mit jeweils einem daran bereits einsatzbereit angekoppelten Cage umfassen. Diese können jeweils in Form eines Sterilsets bereitgestellt werden. Der Chirurg kann dann intraoperativ dasjenige Sterilset auswählen, dessen Cage für die jeweiligen individuellen Anforderungen am geeignetsten ist. Das Instrumentiersegment kann dann am (universell einsetzbaren) Betätigungssegment angekoppelt und verwendet werden.

Das Instrumentiersegment des Positionierinstruments ist nach der Erfindung bevorzugt für den Einmalgebrauch konzipiert. Das Instrumentiersegment kann in diesem Fall mit kostengünstigeren Materialien realisiert werden und muss nicht mit Blick auf die Anforderungen einer wiederholten hygienischen Aufbereitung (Reinigung/Sterilisation) konstruiert sein. Es ist jedoch auch denkbar, dass das Instrumentiersegment zerlegbar und zumindest teilweise für einen Mehrfacheinsatz konzipiert ist. In diesem Fall ist das Instrumentiersegment somit nicht vollständig für den Einmalgebrauch ausgelegt. Durch die hygienische Wiederaufbereitung einzelner Teile, insbesondere des Grundkörpers, des Instrumentiersegments kann eine komplette Neuanschaffung sowie die damit verbundenen Kosten vermieden werden.

Im Gegensatz zum Instrumentiersegment ist das Betätigungssegment nach der Erfindung bevorzugt für den Mehrfachgebrauch konzipiert. So ist dieses bevorzugt mehrmals sterilisierbar und kann zumindest teilweise, bevorzugt vollständig, aus Metall, insbesondere aus Edelstahl bestehen. Ist das Betätigungssegment in seine Einzelteile zerlegbar, so können bei Bedarf Defektteile kostengünstig ausgetauscht werden. Auch kann dadurch dessen Wartung und hygienische Aufbereitung weiter vereinfacht werden.

Gemäß einer besonders bevorzugten Ausführungsform weist der Cage ein Kopfsegment und ein Fußsegment auf, die um eine Schwenkachse relativ zueinander verschwenkbar sind. Die Schwenkachse ist bevorzugt zur Transversalachse des Cages parallel verlaufend ausgerichtet. Dadurch kann der Cage an eine gewünschte Winkelstellung der an das zur Aufnahme des Cages vorgesehenen Bandscheibenfachs angrenzenden Wirbelkörper auf einfache Weise individuell angepasst werden. In der Neutralstellung des Cages sind die Oberseite oder Deckfläche des Kopfsegments und die Unterseite oder Grundfläche des Fußsegments vorzugsweise zueinander parallel oder im Wesentlichen zueinander parallel verlaufend angeordnet.

Im gegeneinander abgewinkelten oder ausgelenkten Zustand des Kopf- und des Fußsegments schließen die Oberseite und die Unterseite gemeinsam einen Winkel α ein. Der Winkel α ist bevorzugt zur Vorderseite des Cages hin offen.

Sind das Fußsegment und das Kopfsegment im Ausgangszustand des Cages gegeneinander frei verschwenkbar, so kann nach dem Positionieren des Cages im Bandscheibenfach beabsichtigt eine (Hyper-) Lordosierung der Wirbelsäule herbeigeführt werden, um den gewünschten Schwenk- oder Anstellwinkel zwischen dem Kopfsegment und dem Fußsegment einzustellen.

Aufgrund der erforderlichen kompakten Baugröße des Cages sind das Kopfsegment und das Fußsegment nach der Erfindung vorteilhaft über zumindest eine Nut-Federverbindung, bevorzugt über zwei solche Nut-Federverbindungen, aneinander geführt und miteinander verbunden. Unter einer Nut-Federverbindung werden in der vorliegenden Anmeldung auch Spundverbindungen verstanden. Mit einer solchen Nut-Federverbindung kann einerseits eine (rein) drehgelenkige Verkopplung der beiden Bauteile auf engstem Raum realisiert werden. Zusätzliche Gelenkbauteile erübrigen sich. Darüber hinaus können die beiden Bauteile durch eine solche Nut-Federverbindung auf einfache Weise unverlierbar miteinander verbunden werden. Dies kommt der Patientensicherheit zugute.

Der Cage weist nach einer bevorzugten Ausführungsform der Erfindung ein Fixierelement auf, mittels dessen das Fußsegment und das Kopfsegment in ihrer jeweiligen Schwenkstellung, d. h. in ihrem jeweiligen Anstellwinkel, relativ zueinander lagefixierbar sind. Das Fixierelement kann insbesondere in Form einer Schraube, beispielsweise einer sogenannten Madenschraube ausgebildet sein. Zur Aufnahme der Fixierschraube dient ein Einschraubbereich des Cages. Der Einschraubbereich kann (anstelle eines Gegengewindes) einfache Riffel oder Rippen aufweisen, die einem form- und kraftschlüssigen Eingriff der Fixierschraube erlauben. Dadurch kann ein sicherer Sitz der Fixierschraube am Cage unabhängig vom Anstellwinkel der beiden Cagesegmente erreicht werden. Die Fixierschraube weist bevorzugt ein Mitnahmeprofil in Form eines Innensechskants oder eines anderen Innenvielzahnprofils auf.

Das Cagepositioniersystem umfasst vorzugsweise ein Drehwerkzeug, mittels dessen die Fixierschraube, bevorzugt vom Betätigungsabschnitt her, betätigbar ist. Das Drehwerkzeug kann nach der Erfindung durch das vorstehend erläuterte erste Arretiermittel gebildet sein. Dadurch kann die Anzahl der erforderlichen Bauteile klein gehalten werden. Dies bietet Kostenvorteile.

Das Betätigungssegment und das Instrumentiersegment sind bevorzugt ineinander steckbar und mittels eines Verriegelungsmittels gegeneinander verriegelbar. Das Verriegelungsmittel kann nach der Erfindung wippenartig ausgeführt sein.

Das Betätigungssegment kann eine Handhabe aufweisen, mittels dessen der bzw. die Manipulatorzinken betätigbar sind. Das Betätigungssegment weist in diesem Fall bevorzugt eine Anzeige auf, anhand derer der jeweilige Drehwinkel des Cages relativ zu seiner Neutralposition bzw. zur Längsachse des Positionierinstruments ablesbar ist.

Der bzw. die vorstehend erläuterten Koppelzinken des Instrumentiersegments können am Grundkörper des Instrumentiersegments insbesondere angeformt sein. Der Grundkörper kann dadurch als sogenanntes CNC-Bauteil (Computerized Numerical Control- Bauteil) oder als Spritzgussteil ausgeführt sein oder im Wege eines 3D-Druckverfahrens erzeugt sein. Der Grundkörper weist bevorzugt mehrere Führungskanäle auf, in denen der/die Manipulatorzinken, der/die Arretiermittel und/oder das Drehwerkzeug verschiebbar geführt sind.

Der Cage besteht nach der Erfindung vorzugswiese zumindest teilweise, bevorzugt vollständig, aus Titan oder einer Titanlegierung oder aus einer Kobalt-Chrom Legierung (CoCr-Legierung). Dadurch kann einerseits ein ausreichend großes Lastaufnahmevermögen sowie auch die erforderliche Biokompatibilität des Cages gewährleistet werden.

Der erfindungsgemäße Cage ist für ein vorstehend erläutertes Cagepositioniersystem vorgesehen und umfasst eine Einstecköffnung für das Instrumentiersegment des Positionierinstruments sowie Kopplungsmittel, die für einen Eingriff jeweils eines der Koppelglieder des Positionierinstruments ausgelegt sind. Der Cage weist vorzugsweise ein Kopfsegment und ein Fußsegment auf, die um eine Schwenkachse relativ zueinander verschwenkbar sind. Die Schwenkachse ist zur Transversalachse des Cages vorzugsweise parallel verlaufend angeordnet.

Die Kopplungsmittel des Cages sind vorzugsweise in Richtung der Transversalachse des Cages voneinander beabstandet am Cage angeordnet.

Das erfindungsgemäße Positionierinstrument ist für ein Cagepositioniersystem vorgesehen und weist bevorzugt ein für einen Einmalgebrauch konzipiertes Instrumentiersegment auf.

Weitere Vorteile der Erfindung ergeben sich aus der Beschreibung und der Zeichnung. Die gezeigten und beschriebenen Ausführungsformen sind nicht als abschließende Aufzählung zu verstehen, sondern haben vielmehr beispielhaften Charakter für die Schilderung der Erfindung.

In der Zeichnung zeigen:
- Fig. 1: ein Cagepositioniersystem mit einem Positionierinstrument mit einem Instrumentiersegment und einem Betätigungssegment sowie mit einem am Positionierinstrument angekoppelten Cage, in einer perspektivischen Ansicht;
- Fig. 2: das Cagepositioniersystem gemäß Fg. 1 in einer teilweise explodierten Darstellung seiner Teile, in einer perspektivischen Ansicht;
- Fig. 3: das Betätigungssegment gemäß Fig. 1 in einer teilweise explodierten Darstellung seiner Teile, in einer perspektivischen Ansicht;
- Fig. 4: einen Detailausschnitt des Betätigungssegments und des Instrumentiersegments des Positionierinstruments gemäß Fig. 1 vor dem reversiblen Verkoppeln der beiden Teile, in einer perspektivischen Ansicht;
- Fig. 5: einen Detailausschnitt des Betätigungssegments und des Instrumentiersegments des Positionierinstruments gemäß Fig. 1, in einer perspektivischen Ansicht;
- Fig. 6: einen Detailausschnitt des Instrumentiersegments des Positionierinstruments gemäß Fig. 1, in einer perspektivischen Ansicht;
- Fig. 7: eine alternative Ausführungsform des Instrumentiersegments des Positionierinstruments gemäß Fig. 1, in einer perspektivischen Ansicht;
- Fig. 8: eine erste Ausführungsform des Cages gemäß Fig. 1 , in einer perspektivischen Ansicht;
- Fig. 9: den Cage gemäß Fig. 8 in einer Seitenansicht;
- Fig. 10: den Cage gemäß Fig. 8 in einer Ansicht auf dessen Grundfläche;
- Fig. 11: eine alternative Ausführungsform des Cages gemäß Fig. 1 in einer perspektivischen Ansicht;
- Fig. 12: einen in sich verschwenkbaren Cage mit einem Fußsegment und einem Kopfsegment, die relativ zueinander in Neutralstellung angeordnet sind, in einer Seitenansicht;
- Fig. 13: den Cage gemäß Fig. 12 im ausgelenkten Zustand des Kopfsegments, in einer Seitenansicht;
- Fig. 14: das Fußsegment des Cages gemäß Fig. 12 mit einem daran angekoppelten Positionierinstrument, in einer Seitenansicht;
- Fig. 15: einen Cage, der zu dem Cage gemäß Fig. 12 ähnlich ist, mit einem daran angekoppelten Positionierinstrument mit zwei Kopplungszinken, die jeweils mit einem Koppelglied versehen sind, wobei die Koppelglieder die in dazu korrespondierende Ausnehmungen des Cages eingreifen;
- Fig. 16: ein Cage-Positioniersystem, dessen Cage relativ zum Positionierinstrument in Neutralstellung angeordnet ist, in einer Draufsicht mit teiltransparenter Darstellung des Cages;
- Fig. 17: das Positioniersystem gemäß Fig. 16 mit gegenüber dem Positionierinstrument verschwenktem Cage, in einer Draufsicht mit teiltransparenter Darstellung des Cages;
- Fig. 18: das Positioniersystem gemäß Fig. 17 in einer perspektivischen Ansicht;
- Fig. 19: das Positioniersystem gemäß Fig. 16 mit stärker verschwenktem Cage, in einer Draufsicht mit teiltransparenter Darstellung des Cages;
- Fig. 20: das Positioniersystem gemäß Fig. 19 mit gegeneinander lagefixierten Fuß- und Kopfsegment des Cages, in einer Draufsicht mit teiltransparenter Darstellung des Cages;
- Fig. 21: das Cagepositioniersystem gemäß Fig. 1 nach dem Implantieren des Cages, in einer perspektivischen Ansicht;
- Fig. 22: das Cagepositioniersystem gemäß Fig. 1 nach dem Implantieren des Cages, in Seitenansicht;
- Fig. 23: das Cagepositioniersystem gemäß Fig. 1 beim Entriegeln des Instrumentierabschnitts, in einer perspektivischen Ansicht;
- Fig. 24: das Cagepositioniersystem gemäß Fig. 23 in einer Seitenansicht;
- Fig. 25: ein Ausführungsbeispiel des Cages gemäß Fig. 1, in einer teilweise geschnittenen Ansicht;
- Fig. 26: ein Blockschaltbild mit einzelnen Verfahrensschritten zum Implantieren eines Cages

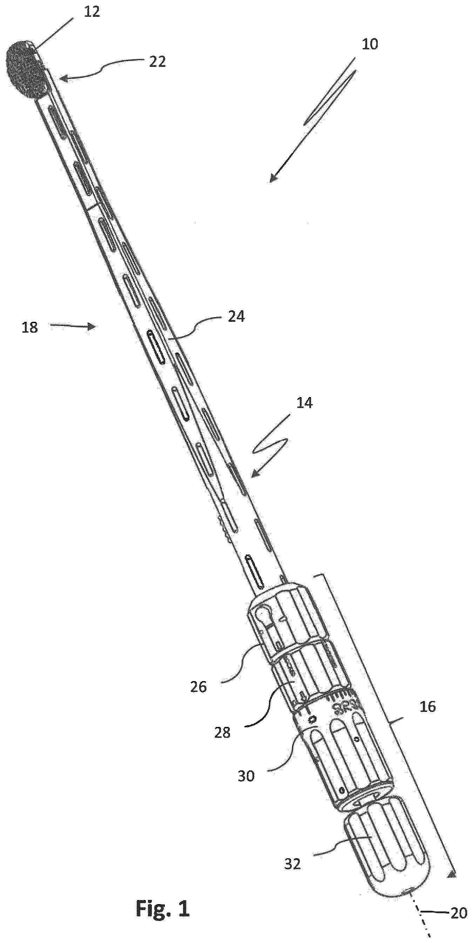

**Fig. 1** zeigt ein Cagepositioniersystem **10** umfassend einen als Bandscheibenersatz dienenden Cage **12** und ein Positionierinstrument **14** zum Implantieren des Cages 12 in einem Bandscheibenfach der Wirbelsäule. Das Positionierinstrument 14 umfasst einen Betätigungssegment **16** und einen Instrumentiersegment **18,** das sich vom Betätigungssegment 16 in Richtung der Längsachse **20** des Positionierinstruments 14 wegerstreckt. Das Instrumentiersegment 18 ist hier mit seinem freien Ende **22** am Cage 12 lösbar angekoppelt. Das Instrumentiersegment 18 umfasst einen mit **24** bezeichneten Grundkörper, der als Hohlkörper ausgeführt ist. Der Grundkörper 24 besteht bevorzugt aus Edelstahl oder aus einem Kunststoff.

Das Betätigungssegment 16 umfasst mehrere Handhaben **26, 28, 30, 32,** deren jeweilige Funktion weiter unten erläutert ist.

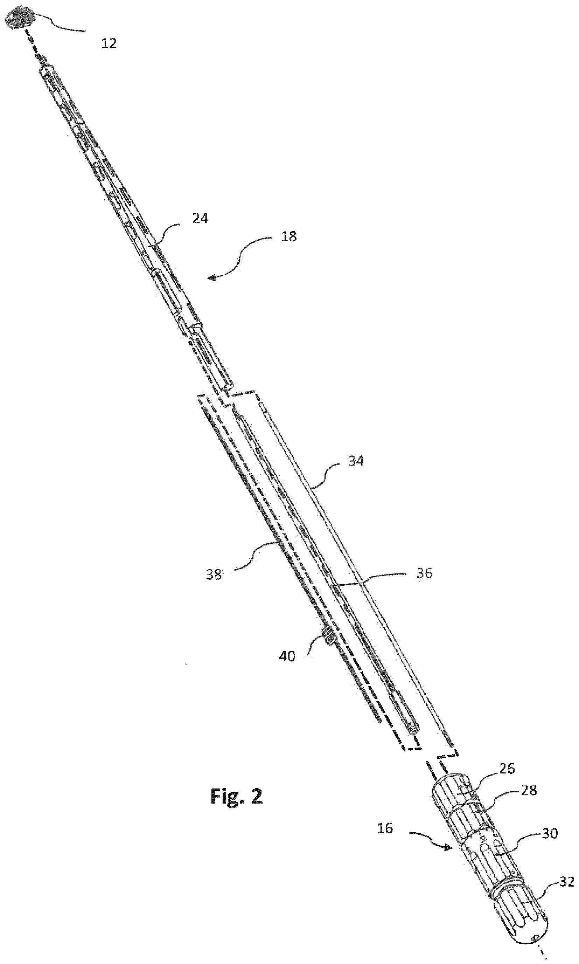

In **Fig. 2** ist das Cagepositioniersystem 10 in einer teilweise explodierten Darstellung seiner Teile gezeigt. Der Cage 12 ist vom Instrumentiersegment 18 abgekoppelt. Innerhalb des Grundkörpers 24 sind ein erstes Arretiermittel in Form eines ersten Arretierschiebers **34,** ein Manipulator **36** und ein dem Manipulator 36 zugeordnetes zweites Arretiermittel in Form eines zweiten Arretierschiebers **38** längsverschieblich geführt. Der zweite Arretierschieber 38 ist mit einer schlittenartigen Handhabe **40** versehen, die mit einer geriffelten Fingerauflage versehen sein kann. Die Handhabe 40 ist am Grundkörper24 längsverschieblich gelagert.

Das Instrumentiersegment 18 und das Betätigungssegment 16 sind hier jeweils als getrennt handhabbare und vormontierte Baueinheiten oder Instrumentenmodule ausgebildet, die für den Betriebseinsatz des Positionierinstruments 14 miteinander lösbar verkoppelbar sind.

Der Instrumentierabschnitt 18 ist vorzugsweise für den Einmalgebrauch ausgelegt. Das Betätigungssegment 16 ist demgegenüber bevorzugt für den Mehrfachgebrauch konzipiert. Das Betätigungssegment 16 besteht zumindest teilweise, bevorzugt insgesamt, aus Metall, insbesondere Edelstahl. Alternativ kann das Betätigungssegment aus einem qualitativ besonders hochwertigen und für wiederholte Sterilisationsmaßnahmen geeigneten Kunststoff bestehen.

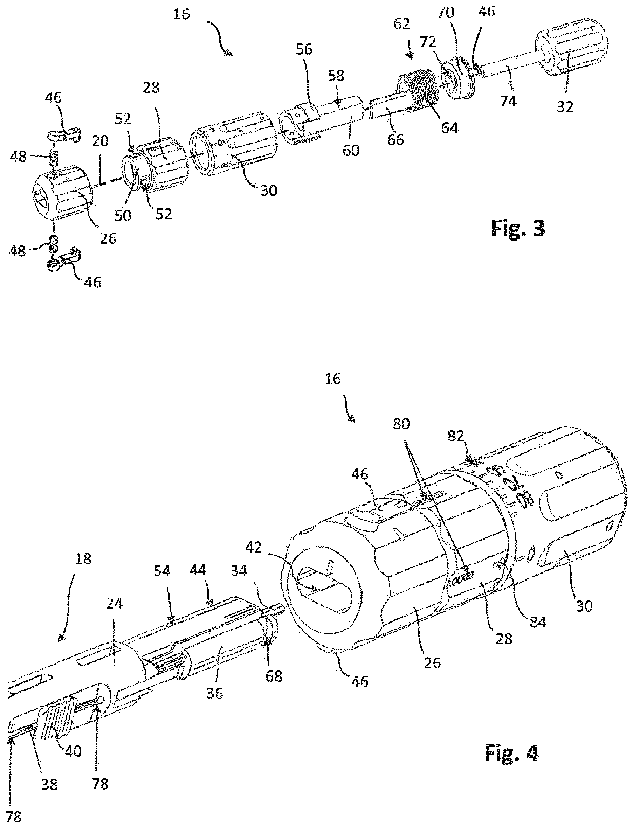

In **Fig. 3** ist das Betätigungssegment 16 in einer teilweise explodierten Darstellung seiner Einzelteile gezeigt.

Die erste Handhabe 26 ist in axialer Richtung distal am Betätigungssegment 16 angeordnet. Diese erste Handhabe 26 dient dem in die zweite Handhabe 28 einzuführenden Instrumentiersegment 18 als Arretierknebel. Die erste Handhabe 26 weist hierzu eine stirnseitige Einführöffnung **42** für das Instrumentiersegment 18 auf. Die Einführöffnung 42 weist eine zum proximalen Endabschnitt **44** des Instrumentiersegments 18 korrespondierende Querschnittsform auf. Dadurch kann das Instrumentierabschnitt von der ersten Handhabe drehfest umschlossen werden. An der ersten Handhabe 26 sind Verriegelungsmittel **46** angeordnet, die gemäß Fig. 3 als wippenartig gelagerte Sperrklinken ausgeführt sein können. Die Verriegelungsmittel 46 sind im montierten Zustand durch Federelemente **48** vorgespannt in ihrer jeweiligen Verriegelungsstellung gehalten und demzufolge jeweils gegen die Kraft eines der Federelemente 48 in ihre Entriegelungsstellung überführbar. Die Federelemente 48 können gemäß Fig. 3 jeweils als Druckfeder ausgebildet sein. Der erste Handhabe 26 ist auf einem Flansch **50** der als Griffabschnitt dienenden zweiten Handhabe 28 des Betätigungssegments 16 rotierbar gelagert. Der Flansch 50 weist zur axialen Lagesicherung der ersten Handhabe an der zweiten Handhabe Eingriffsausnehmungen **52** für die Verriegelungsmittel 46 auf.

Gemäß **Fig. 4** weist der proximale Endabschnitt 44 des Instrumentiersegments 16 eine Nut **54** auf. Die zweite Handhabe 28 weist innenseitig einen zur Nut 54 komplementären Verriegelungssteg auf, der in **Fig. 3** aus Darstellungsgründen nicht zu erkennen ist. Der Verriegelungssteg ist durch eine Rotation des ersten Handhabe 26 mit dem über die Einführöffnung 42 bis auf Anschlag in die erste und zweite Handhabe 26, 28 eingesteckten Instrumentiersegment 18 relativ zur zweiten Handhabe 28 in die Nut 54 bewegbar. Dadurch ist das Instrumentiersegment 18 an der zweiten Handhabe 28 in axialer Richtung (reversibel) lagefixierbar. Ein unbeabsichtigtes Lösen des Instrumentierabschnitts 18 vom Betätigungsabschnitt 16 ist durch die in die Eingriffsausnehmungen 52 eingreifenden Verriegelungsmittel 46 gewährleistet

An die zweite Handhabe 28 schließt sich in axialer Richtung eine dritte Handhabe 30 an. Die dritte Handhabe 30 ist im montierten Zustand des Betätigungssegments 16 relativ zur zweiten Handhabe 28 um die Längsachse 20 rotierbar. Innerhalb der zweiten und dritten Handhabe 28, 30 ist eine Lagerhülse **56** mit einem Lagerstift **58** angeordnet, der sich hier von der Lagerhülse 56 in axialer Richtung nach proximal wegerstreckt. Die Lagerhülse 56 ist mit der zweiten Handhabe 28 verstiftet. Der Lagestift 58 weist einen abgeflachten Umfangsbereich **60** auf. Mit **62** ist eine Bewegungshülse bezeichnet, die im montierten Zustand des Betätigungssegments 16 mit ihrem Außengewinde **64** mit der dritten Handhabe 30 im gegenseitigen Gewindeeingriff steht. Die Bewegungshülse 64 weist einen teilumfänglich abgeflachten Stößel **66** auf, der im montierten Zustand des Positionierinstruments am Lagerstift 58 der Lagerhülse 56 abschnittsweise anliegt und längsverschieblich geführt ist.

Am Stößel 66 der Bewegungshülse 62 ist innenseitig ein Mitnehmer angeordnet, der von der Bewegungshülse 62 in radialer Richtung nach innen wegragt. Der Mitnehmer greift im am Betätigungssegment 16 angekoppelten Zustand des Instrumentiersegments 18 in eine Haltenut **68** des Manipulators 36 ein, um diesen mit der Bewegungshülse 62 in axialer Richtung bewegungszukoppeln. Die Bewegungshülse 62 ist mittels eines Deckelrings **70** innerhalb der dritten Handhabe 30 gehalten angeordnet. Der Deckelring 70 kann mit der dritten Handhabe 30 verstiftet sein. Der Deckelring 70 weist eine axiale Durchgangsöffnung **72** auf, über die ein als Drehknebel ausgeführte vierte Handhabe 32 mit einem Mitnehmerstift **74** bis in die zweite Handhabe 28 des Betätigungssegments 16 einsteckbar ist. Der Mitnehmerstift 74 weist an seinem distalen Ende ein Mitnehmerprofil **76,** hier ein Innenmehrkantprofil, auf. Das Innenmehrkantprofil dient der Aufnahme des in Fig. 2 und 4 mit 34 bezeichneten ersten Arretierschiebers, der sich im Montagezustand des Positionierinstruments 14 einenends in das Betätigungssegment 16 hineinerstreckt.

In Fig. 4 ist die am Instrumentierabschnitt angeordnete fünfte Handhabe 40 des Positionierinstruments gut zu erkennen. Die fünfte Handhabe ist in axialer Richtung zwischen zwei Endpositionen **78** hin- und herbewegbar. Die Funktion der fünften Handhabe 40 ist weiter unten erläutert.

Die zweite Handhabe 28 weist erste Markierungen **80** auf, über die der Verriegelungszustand der ersten Handhabe 26 mitsamt dem Instrumentiersegment 18 relativ zur zweiten Handhabe 28, hier deren Verriegelungsmittel 46, ablesbar ist. Die dritte Handhabe 30 weist zweite Markierungen **82** mit Winkelinformationen auf, die relativ zu einer Bezugsmarkierung **84** der zweiten Handhabe abzulesen sind.

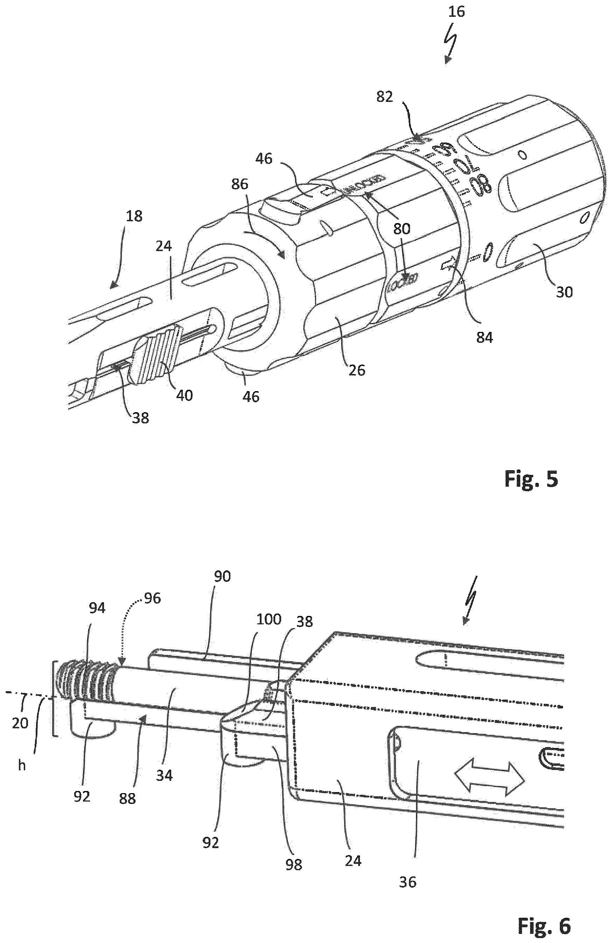

In **Fig. 5** ist das Instrumentiersegment 18 in seiner in das Betätigungssegment 16 eingesteckten vorgegebenen Montageposition gezeigt. Das Instrumentiersegment 18 kann durch Drehen der ersten Handhabe 26 in Pfeilrichtung **86** im Betätigungssegment 16 verriegelt, d. h. in axialer Richtung lagefixiert, werden.

In **Fig. 6** ist ein vergrößerter Detailausschnitt des distalen Endes des Instrumentiersegments 18 gezeigt. Der Grundkörper 24 des Instrumentiersegments 18 weist einenends einen Kopplungszinken **88** auf, der sich in axialer Richtung vom Grundkörper 24 wegerstreckt. Der Kopplungszinken 88 kann einen abgewinkelten Randbereich **90** aufweisen, um eine auseichende Beigesteifigkeit des Kopplungszinkens 88 zu gewährleisten. Der Kopplungszinken 88 weist in diesem Fall eine L-förmige oder im Wesentlichen L-förmige Querschnittsform auf.

Am freien Endabschnitt des Kopplungszinkens 88 ist ein Koppelglied **92** angeordnet. Das Koppelglied 92 erstreckt sich vom Kopplungszinken 88 in radialer Richtung seitlich weg. Vorliegend ist das Koppelglied 92 kreiszylinderförmig ausgeführt. Am Kopplungszinken 88 liegt der im Zusammenhang mit Fig. 5 erläuterte erste Arretierschieber 34 (oberseitig) an. Der erste Arretierschieber 34 ist durch den abgewinkelten Randbereich 90 des Kopplungszinkens 88 seitlich geschient.

Am freien Ende des ersten Arretierschiebers 34 kann ein Fixierelement **94,** hier beispielhaft in Form einer Madenschraube, angeordnet sein. Das Fixierelement 94 kann dabei ein nicht näher dargestelltes Mitnahmeprofil, etwa in Form eines Innensechskants aufweisen, in den der Arretierschieber 34 (mit einem Außensechskant oder sogenannten Innensechskantschlüssel) drehfest eingreift. Alternativ kann die Fixierelement 94 auch einstückig mit dem Arretierschieber 34 verbunden sei. In diesem Fall sind der Arretierschieber 34 und das Fixierelement 94 vorteilhaft über eine Sollbruchstelle **96** miteinander verbunden, um den Arretierschieber 34 nach dem Platzieren der Fixierelements 94 vom Fixierelement 94 trennen zu können. Vorstellbar ist auch, dass das Fixierelement 94 am ersten Arretierschieber 34 festgeklebt ist oder auf dem Arretierschieber 34 im Presssitz gehalten angeordnet ist. Der Arretierschieber 34 kann alternativ ein Werkzeugprofil, beispielsweise einen Innensechskantschlüssel aufweisen, das in ein dazu korrespondierendes Mitnahmeprofil (=Antriebsprofil) des Fixierelements 94 lösbar eingreift. Das Fixierelement 94 ist vorzugsweise als eine Schraube ausgebildet.

Der Manipulator 36 weist einenends einen mit **98** bezeichneten Manipulatorzinken auf. Der Manipulatorzinken 98 weist in einer zum Kopplungszinken 88 entsprechenden Weise ein Koppelglied 92 auf, das sich vom Manipulatorzinken 98 in radialer Richtung, d. h. wegerstreckt. Die Koppelglieder 92 der beiden Zinken können sich gemäß Fig. 6 vom jeweiligen Zinken gleichsinnig oder gemäß einer in der Zeichnung nicht näher dargestellten Ausführungsform in einander entgegengesetzte Richtungen vom jeweiligen Zinken wegerstrecken.

Dem Manipulator 36 ist ein zweiter Arretierschieber 38 zugeordnet. Der zweite Arretierschieber 38 kann an dem Manipulatorzinken 98 unmittelbar anliegen und sich an diesem in einer radialen Richtung abstützen. Das freie Ende des Arretierschiebers 38 kann eine Einführschräge **100** aufweisen. Die kumulative Bauhöhe **h** des Kopplungszinkens 88 und des dem Kopplungszinken 88 zugeordneten ersten Arretierschiebers 34 sowie die kumulative Bauhöhe h des Manipulatorzinkens 98 und des zugeordneten zweiten Arretierschiebers 38 sind in Fig. 6 gut zu erkennen.

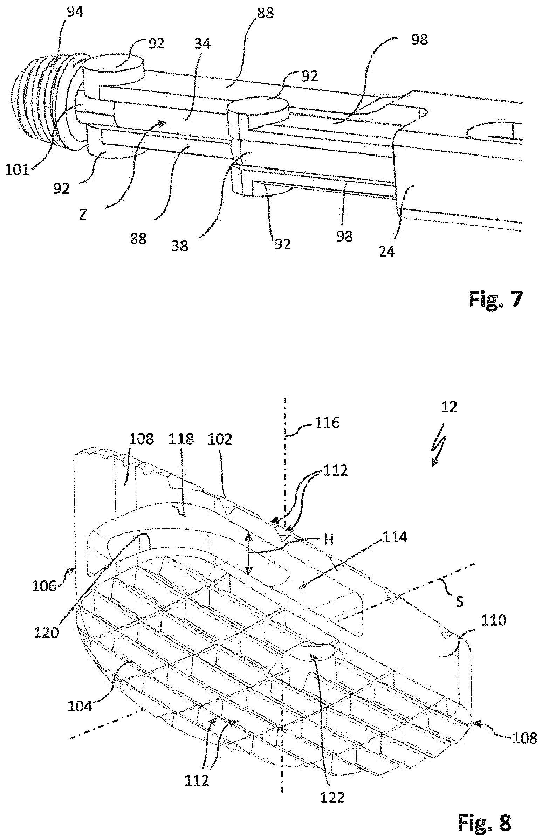

Das Positionierinstrument 14 kann gemäß der einer in **Fig. 7** ausschnittsweise gezeigten Ausführungsform einen zweiten Kopplungszinken 88 und/oder einen zweiten Manipulatorzinken 98 aufweisen. Der zweite Kopplungszinken 88 ist zum ersten Kopplungszinken 88 parallel oder im Wesentlichen parallel verlaufend angeordnet. dadurch ist zwischen den beiden Kopplungszinken 88 ein Zwischenraum **Z** ausgebildet, in den der erste Arretierschieber 34 in Richtung der Längsachse 20 des Positionierinstruments 14 vorschiebbar ist. Die Koppelglieder 92 der beiden Kopplungszinken 88 weisen vom jeweiligen Kopplungszinken 88 in einander entgegengesetzte Richtungen weg. Wird der erste Arretierschieber 34 zwischen den beiden Kopplungszinken 88 in axialer Richtung nach vorn geschoben, so sperrt dieser ein Approximieren der beiden Kopplungszinken 88. Auch kann der erste Arretierschieber 34 die Kopplungszinken 88 ggf. (geringfügig) auseinanderspreizen. Der Arretierschieber 34 greift gemäß Fig. 7 reversibel mit einem Werkzeugprofil **101,** hier einem sogenannten Innensechskantschlüssel in ein dazu korrespondierendes Mitnahme- oder Antriebsprofil der Fixierelements 94. Die beiden Kopplungszinken 88 sind am Grundkörper 24 vorzugsweise angeformt und an diesem flexibel angelenkt.

Der zweite Manipulatorzinken 98 ist zum ersten Manipulatorzinken 98 parallel oder im Wesentlichen parallel verlaufend angeordnet. Dadurch ist zwischen den beiden Manipulatorzinken 98 ein Zwischenraum Z ausgebildet, in den der zweite Arretierschieber 38 vorschiebbar ist. Die Koppelglieder 92 der beiden Manipulatorzinken 98 weisen vom jeweiligen Manipulatorzinken 98 in einander entgegengesetzte Richtungen weg. Wird der Arretierschieber 38 zwischen den beiden Manipulatorzinken 98 in axialer Richtung nach vorn geschoben, so sperrt dieser ein Approximieren der beiden (mit dem Cage verkoppelten) Manipulatorzinken 98. Auch kann der zweite Arretierschieber 38 die Manipulatorzinken 98 ggf. (geringfügig) auseinanderspreizen, so dass diese ggf. zum freien Ende hin divergieren.

In den **Fign. 8** bis **10** ist eine erste Ausführungsform des Cages 12 gemäß Fig. 1 gezeigt. Der Cage 12 weist hier eine nierenförmige Grundform auf. Eine Deckfläche (Oberseite) des Cages ist mit **102** und seine gegenüberliegend angeordnete Grundfläche (Unterseite) ist mit **104** bezeichnet. Die im implantierten Zustand des Cages 12 in Richtung seiner Sagittalachse **S** nach vorn weisende Seitenfläche **106** ist über zwei einander gegenüberliegende Seitenflächen **108** mit der rückseitigen Seitenfläche **110** verbunden. Die Deckfläche 102 und die Grundfläche 104 des Cages 12 weisen eine makroskopische Oberflächenstrukturierung **112** auf, durch die einen verbesserter knöcherner Reibschluss sowie eine verbesserte Osseointegration des im Bandscheibenfach implantierten Cages 12 ermöglichen werden soll.

Der Cage 12 weist eine mit **114** bezeichnete Einstecköffnung für das Instrumentiersegment 18 des Positionierinstruments 14 auf. Die Einstecköffnung 114 erstreckt sich in Umfangsrichtung von einer seiner Seitenflächen 108 bis in die rückseitige Seitenfläche 110. Eine lichte Höhe der Einstecköffnung 114 ist mit **H** bezeichnet. Die Einstecköffnung 114 ist in Richtung der Vertikalachse **116** des Cages oberseitig durch eine Deckenwandung **118** und unterseitig durch eine Bodenwandung **120** begrenzt.

Die Bodenwandung 120 ist mit einem ersten Kopplungsmittel **122** versehen, das Kopplungsmittel 122 ist hier in Form einer Ausnehmung für den Eingriff des Koppelglieds 92 des Kopplungszinkens 88 des Positionierinstruments 14 ausgeführt. Die Deckenwandung 118 der Einstecköffnung 114 ist mit einem zweiten Kopplungsmittel **124** in Form einer nichtlinearen Ausnehmung versehen, die dem Eingriff des Koppelglieds 92 des Manipulatorzinkens 98 dient.

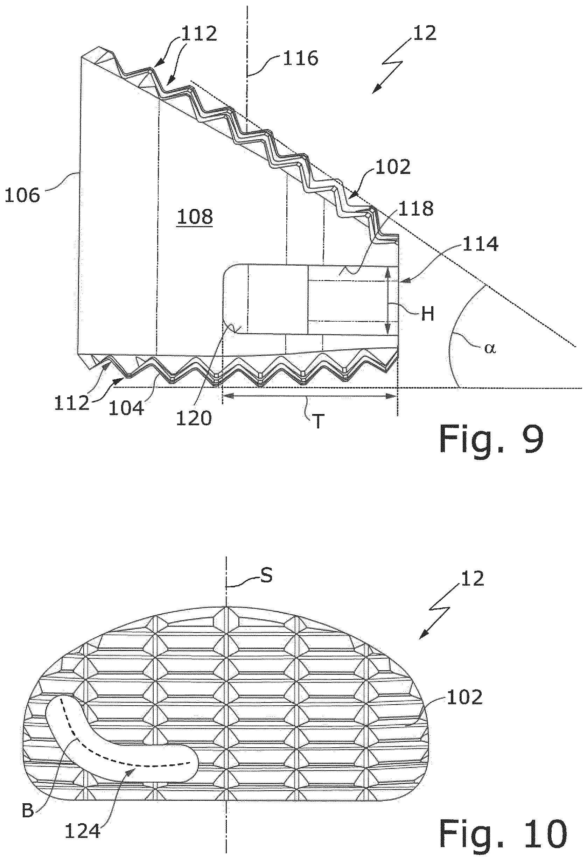

Gemäß **Fig. 9** sind die Deckfläche 102 und die Grundfläche 104 des Cages 12 zueinander unter einem festen Winkel **α** zueinander schräg verlaufend angeordnet. Dadurch weist der Cage 12 eine dreieckige Querschnittsform auf. Die Einstecköffnung 114 weist eine Tiefe **t** auf, die ungefähr der Hälfte der sagittalen Erstreckung des Cages 112 entspricht.

Es versteht sich, dass die Orientierung der Koppelglieder 92 des Kopplungszinkens 88 und des Manipulatorzinkens 98 sowie die Anordnung des jeweilig korrespondierenden Kopplungsmittels 122, 124 des Cages 12 aufeinander abgestimmt sind.

So kann das Kopplungsmittel 122 für das eine Koppelglied 92 des Manipulatorzinken 88 auch an der Bodenwandung 120 der Einstecköffnung 114 angeordnet bzw. ausgebildet sein. In dazu entsprechender Weise kann das Kopplungsmittel 124 für das eine Koppelglied 92 des Kopplungszinkens 88 an der Deckenwandung 118 der Einstecköffnung ausgebildet sein. Durch das zweite Kopplungsmittel 124 ist eine nichtlineare Bewegungsbahn **B** (Fig. 10) für das Koppelglied des Manipulatorzinkens 98 gebildet, entlang derer dessen Koppelglied 92 entlangführbar ist..

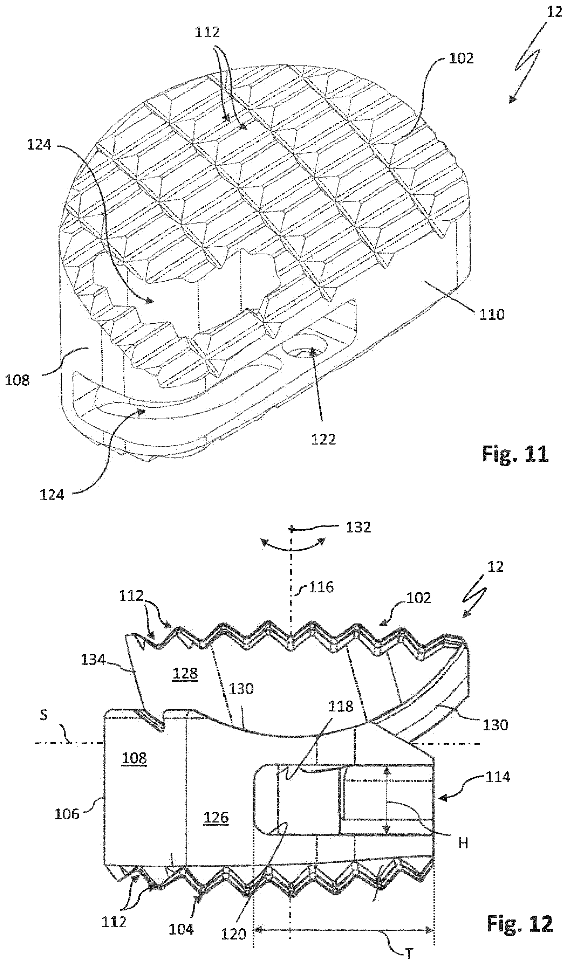

Weist das Positionierinstrument 14 (Fig. 1) zwei Manipulatorzinken 98 auf, so weist der Cage 12 dazu korrespondierend zwei zweite Kopplungsmittel 124 für den Manipulatorzinken 98 auf, von denen eines an/in der Deckenwandung 118 und das andere an/in der Bodenwandung 120 der Einstecköffnung 114 angeordnet sind, wie dies beispielhaft in Fig. 11 gezeigt ist.

In den Figuren **12** bis **13** ist ein weiteres Ausführungsbeispiel des Cages 12 eines Cagepositioniersystems 10 gemäß Fig. 1 gezeigt. Dieser Cage 12 weist ein Fußsegment **126** und ein Kopfsegment **128** auf, die miteinander gelenkig verbunden sind. Dadurch können durch den Cage unterschiedliche Lordosewinkel der Wirbelsäule abgebildet werden. In Fig. 12 ist der Cage 12 in seiner Neutralstellung gezeigt. In der Neutralstellung sind die Deckfläche 102 und die Grundfläche 104 im Wesentlichen zueinander parallel verlaufend angeordnet, wenngleich die Deckfläche 102 des Kopfsegments 128 und die Grundfläche 104 des Fußsegments 126 jeweils nach außen leicht gewölbt ausgeführt sind.

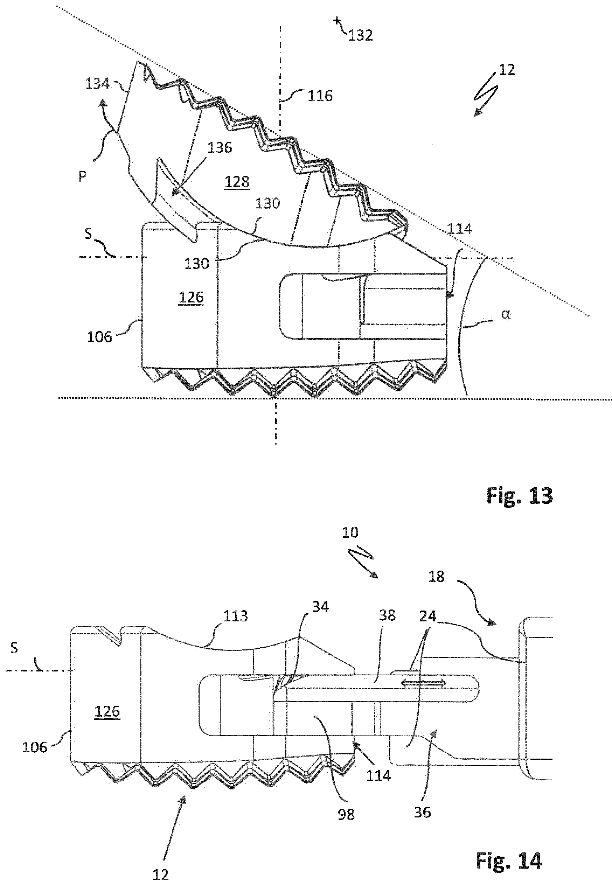

Das Fußsegment 126 und das Kopfsegment 128 weisen einander zuweisende Gelenkflächen **130** auf, über die das Fußsegment 126 und das Kopfsegment 128 aneinander anliegen. Die Gelenkflächen 130 sind jeweils in sagittaler Richtung des Cages 12 gewölbt ausgeführt. Die beiden Segmente 126, 128 sind um eine mit **132** bezeichnete Schwenkachse relativ zueinander schwenkbar. Die Krümmung der beiden Gelenkflächen 130 ist derart, dass die Schwenkachse 132 weit außerhalb des Cages angeordnet ist. Mit einem steigendem Anstellwinkel α zwischen dem Kopfsegment 128 und dem Fußsegment 126 wird die anteriore Kante **134** des Kopfsegments in Richtung der Sagittalachse S des Cages 12 über die vordere Seitenfläche 106 des Cages 12 hinausbewegt, wie dies in Fig. 13 mit dem Pfeil **P** verdeutlicht ist. Dadurch kann der Cage 12 nicht nur variabel an unterschiedliche Winkellagen angrenzender Wirbelkörper angepasst werden, sondern auch eine für die Lordosierung der lumbalen Wirbelsäule verbesserte Abstützung der Wirbelkörper ermöglicht werden.

Das Kopfsegment 126 und das Fußsegment 124 des Cages 12 sind vorzugsweise über zumindest eine Nut-Federverbindung **136,** hier rein beispielhaft über zwei Nut-Federverbindungen 136, aneinander geführt und unverlierbar aneinander gehalten. Dadurch erübrigen sich separate Gelenkbauteile.

In **Fig. 14** ist das Fußsegment 126 des Cages 12 gemäß den Figuren 12 und 13 mit einem daran angekoppelten Positionierinstrument 14 in einer ausschnittsweisen Darstellung gezeigt. Das Positionierinstrument 14 weist ein Instrumentiersegment gemäß Fig. 6 auf. Der Instrumentiersegment 18 weist nur einen Kopplungszinken (in Fig. 14 darstellungsbedingt nicht dargestellt) und nur einen Manipulatorzinken 98 auf.

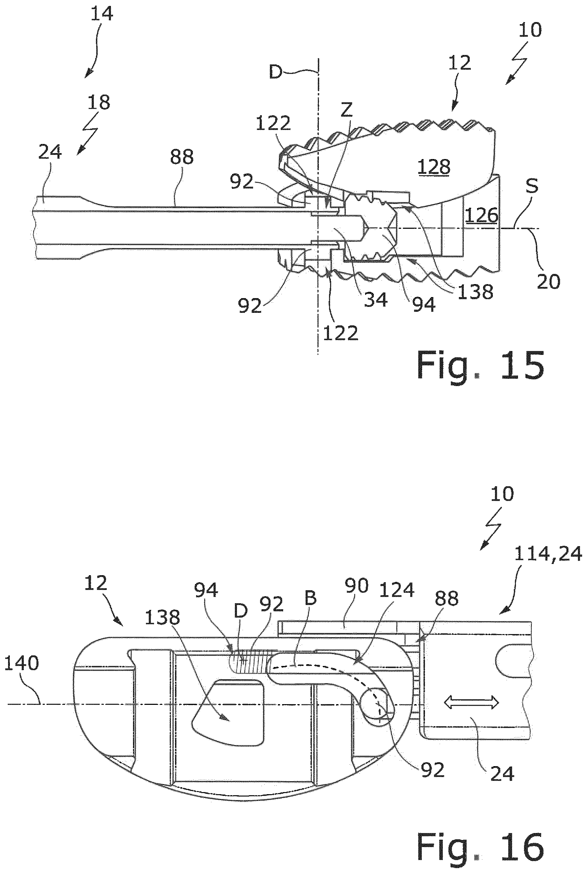

In **Fig. 15** ist ein Cage gemäß Fig. 12 mit einem angekoppelten Positionierinstrument 14 gezeigt. Das Positionierinstrument weist hier zwei Kopplungszinken 88 auf, die mit ihrem jeweiligen Koppelglied 92 in jeweils ein als Bohrung ausgeführtes erstes Kopplungsmittel 122 des Cages 12 formschlüssig eingreifen. Der den beiden Kopplungszinken 88 zugeordnete erste Arretierschieber 34 ist in seiner (distalen) Arretierstellung angeordnet. Die beiden Kopplungszinken 88 sind dadurch in ihrer in die Arretiermittel eingreifenden Ankoppelstellung arretiert.

Die Fixierelement 94 ist noch am ersten Arretierschieber fixiert und kann in den Einschraubbereich 138 des Fußsegments 126 und des Kopfsegments 128 eingeschraubt werden, um diese nach Einstellen eines jeweils gewünschten Anstellwinkels α (Fig. 13) relativ zueinander festzusetzten.

### Funktionsbeschreibung

In **Fig. 16** ist das Positioniersystem 10 mit einem Cage 12 und einem daran angekoppelten Positionierinstrument 14 ausschnittsweise in einer teiltransparenten Draufsicht gezeigt. Mit **140** ist die Transversalachse des Cages bezeichnet, die zur Vertikalachse 116 sowie zur Sagittalachse S orthogonal verlaufend ausgerichtet ist. Der Cage ist mit seiner Transversalachse 140 in seiner neutralen Schwenkstellung um die Drehachse **D** relativ zur Längsachse 22 des Positionierinstruments bzw. dem/den Kopplungszinken 88 des Positionierinstruments 14 angeordnet. Die nichtlineare Bewegungsbahn B für das Koppelglied des Manipulatorzinkens ist gut zu erkennen.

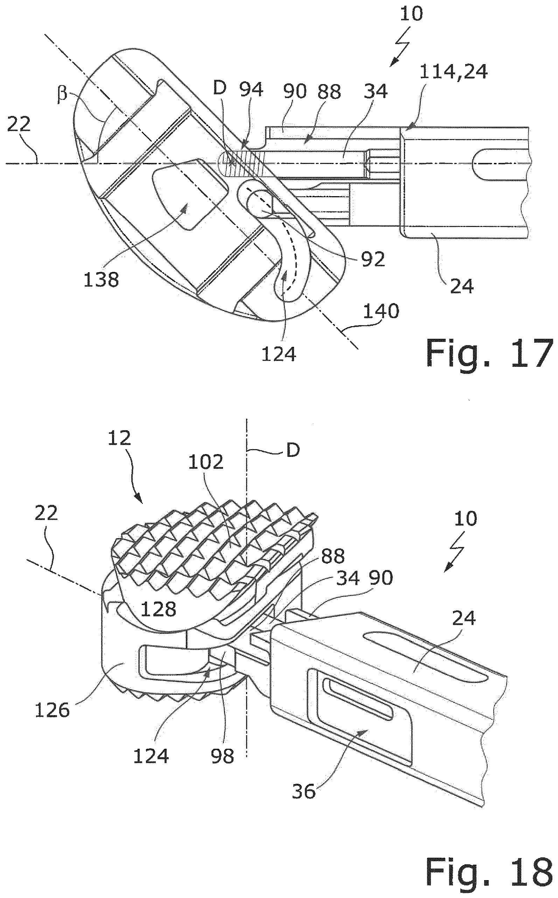

In den **Fig. 17** und **18** ist der Cage 12 mit seiner Transversalachse 140 um einen Schwenkwinkel **β** von ungefähr 45° relativ zur Längsachse 22 des Positionierinstruments 14 verschwenkt angeordnet. Im Vergleich zu der Neutralstellung des Cages 12 (Fig. 16) ist der Manipulator 36 gemeinsam mit seinem Manipulatorzinken 98 in axialer Richtung nach distal verschoben angeordnet.

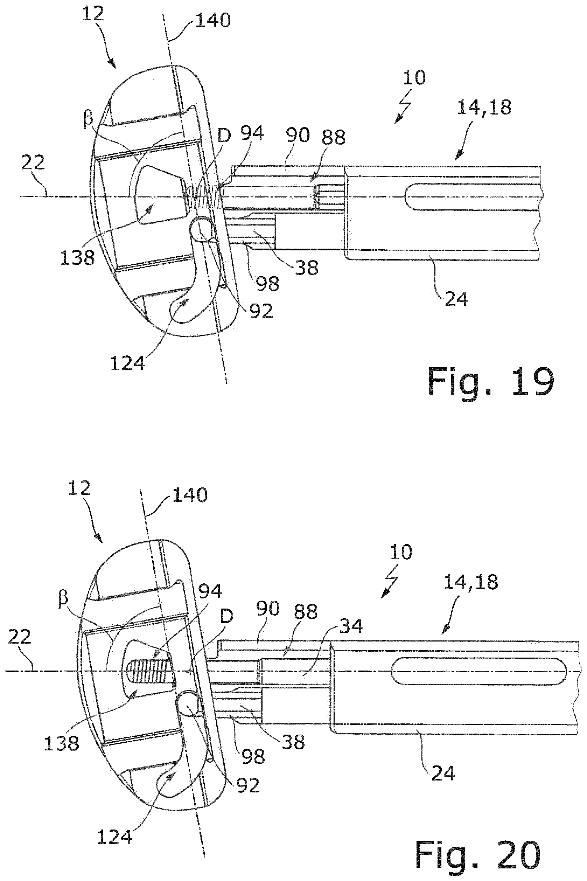

Gemäß **Fig. 19** schließt der Cage 12 mit seiner Transversalachse 140 einen Winkel β von ungefähr 80° mit der Längsachse 22 des Positionierinstruments bzw. dem Kopplungszinken ein. Das kreiszylinderförmige Koppelglied 92 des Manipulatorzinkens 98 liegt am Cage im Endbereich des als nichtlineares Langloch ausgeführten zweiten 124 Kopplungsmittels des Cages 12 an. Dadurch ist ein weiteres Schwenken des Cages 12 um die Drehachse D unterbunden.

In **Fig. 20** ist das als Madenschraube ausgeführte Fixierelement 94 in den Einschraubbereich 138 des Cages 12 eingeschraubt. Das Fußsegment 126 und das Kopfsegment 128 sind dadurch in ihrem Anstellwinkel α relativ zueinander arretiert.

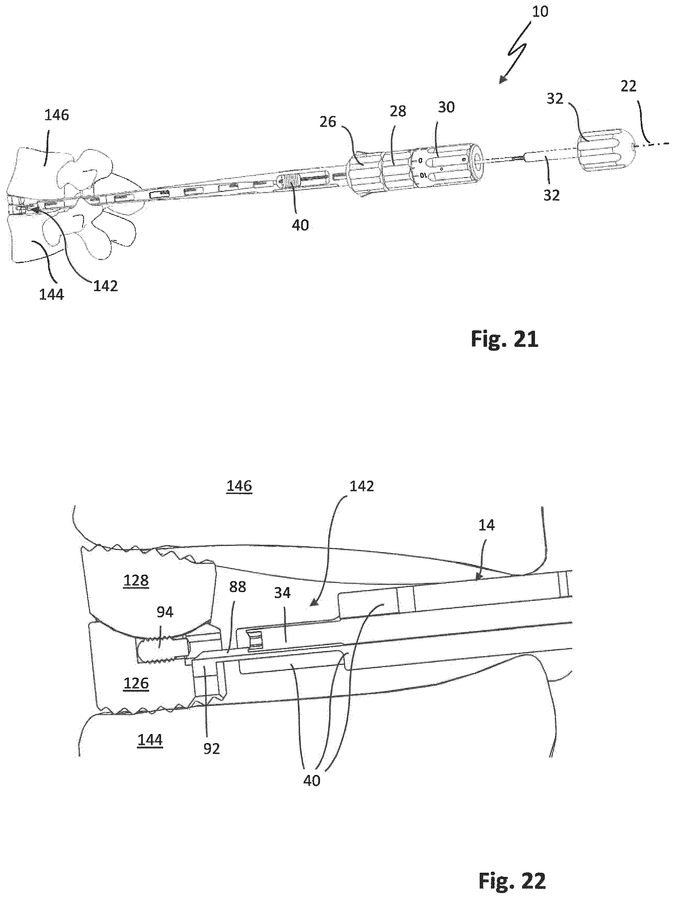

In **Fig. 21** ist der Cage 12 ist im Bandscheibenfach 142 zwischen zwei physiologischer Weise unmittelbar aufeinanderfolgenden der fünf menschlichen Lendenwirbelkörper positioniert. Das Fixierelement 94 wurde mittels der vierten Handhabe 32 des Betätigungssegments 16 des Positionierinstruments in seine Fixierstellung überführt, d.h. hier in den Einschraubbereich 138 des Cages 10 eingeschraubt. Die vierte Handhabe 32 kann hiernach aus dem restlichen Betätigungssegment 16in axialer Richtung herausbewegt werden. Dadurch ist der Kopplungszinken 88 durch den ersten Arretierschieber 34 nicht mehr in seiner Ankoppelstellung am Cage 12 gesichert. Zugleich ist der als Drehwerkzeug dienende Arretierschieber 34 von dem Fixierelement 94 diskonnektiert, wie dies in **Fig. 22** näher gezeigt ist.

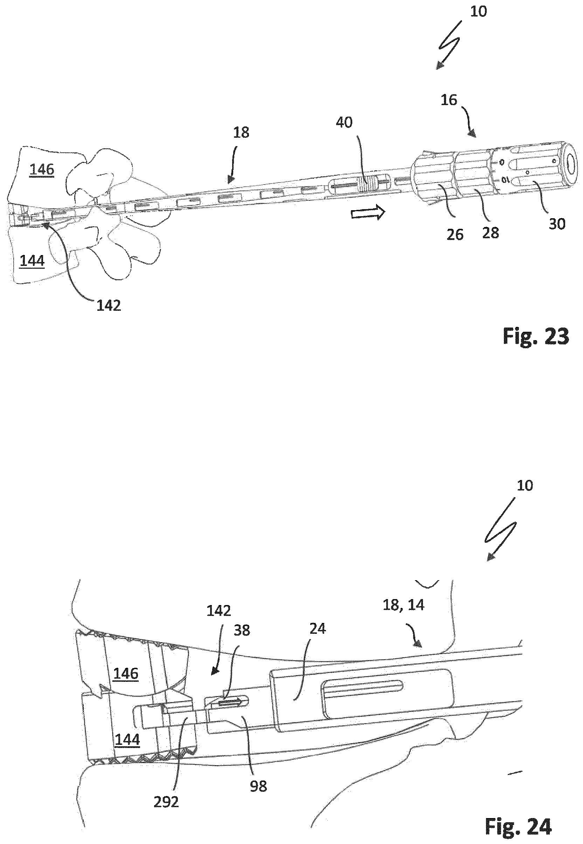

In **Fig. 23** ist die Handhabe 40 des Instrumentiersegments 18 in axialer Richtung nach proximal verschoben. Dadurch ist der mit der Handhabe 40 bewegungsgekoppelte zweite Arretierschieber 38 des Manipulatorzinkens 98 in seine das Diskonnektieren des Manipulatorzinkens 98 vom Cage freigebende Freigabestellung überführt, wie dies in **Fig. 24** gezeigt ist.

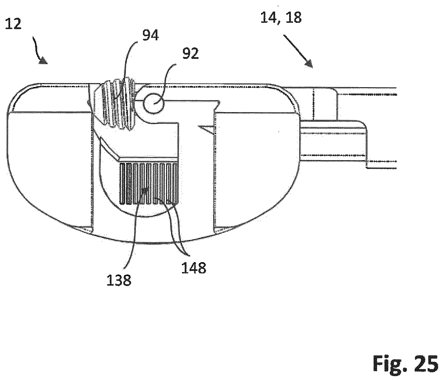

In **Fig. 25** ist ein am Positionierinstrument 14 angekoppelter gelenkig verstellbarer Cage 12 gezeigt. Der Einschraubbereich 138 des Cages 12 kann Riffel **148** aufweisen, die hier an der Deckenwandung 118 und der Bodenwandung 120 der Einstecköffnung 114 angeordnet sind. Die Riffel 148 können auch zumindest teilweise eine von der in Fi. 25 gezeigten linearen Formgebung abweichende Formgebung aufweisen.

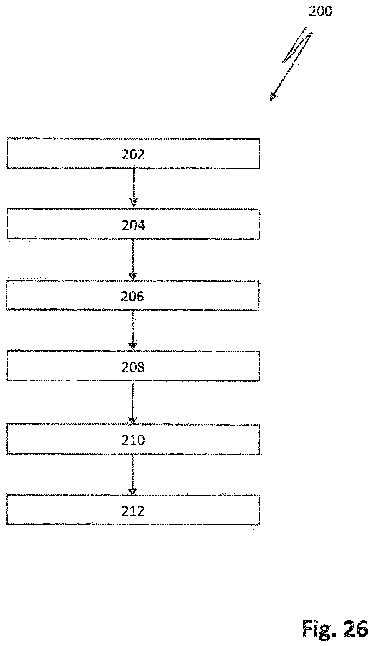

Die Funktionsweise des Cagepositioniersystems 10 wird nachfolgend unter zusätzlicher Bezugnahme auf die in **Figur 26** wiedergegebenen Schritte des Verfahrens **200** zum Implantieren des Cages 12 erläutert.

Zum Implantieren des Cages 12 im zwischen zwei Wirbelkörpern 144, 146 angeordneten Bandscheibenfach 142 der, bevorzugt lumbalen Wirbelsäule, wird das Positionierinstrument 14 in einem ersten Schritt **202** mit seinem/seinen Kopplungszinken 88 und seinem/seinen Manipulatorzinken 98 des Instrumentierabschnitts 18 am Cage 12 angekoppelt.

In einem weiteren Schritt **204** wird der am Cage angekoppelte Instrumentierabschnitt 18 durch Überführen des ersten Arretierschiebers 34 in seine Arretierstellung in seiner am Cage 12 angekoppelten Stellung gesichert. Das Instrumentiersegment 18 kann mit dem Cage 12 derart vormontiert in Form eines Sterilsets bereitgestellt werden.

In einem weiteren Schritt **206** wird das Betätigungssegment 16 am Instrumentiersegment 18 von einer Bedienperson angekoppelt. Dazu wird das Instrumentiersegment 18 in Teilschritt **206a** in die Einführöffnung (vgl. Fig. ) des Betätigungsabschnitts 16 bis zum Anschlag eingeführt. Die Verriegelungsmittel 46 (= Sperrklinken) werden in Teilschritt **206b** entriegelt und der Manipulator 36 des Instrumentiersegments 18 durch dessen rotatorische Verstellung um die Längsachse 22 mit der dritten Handhabe 30 in Eingriff gebracht. In Teilschritt **206c** werden die Verriegelungsmittel 46 in ihre Verriegelungsstellung überführt und so das Instrumentiersegment 18 am Befestigungssegment 16 drehfest und in axialer Richtung lagefixiert festgesetzt.

Das Cagepositioniersystem 10 ist damit einsatzbereit.

Der Cage 12 kann nun in einem weiteren Schritt **208** Richtung seiner Transversalachse 140 in den Operationssitus eingeführt und in das mit dem Cage 12 zu versorgende Bandscheibenfach 142 der Wirbelsäule eingeführt werden.

Durch - hier beispielhaft rotatorisches - Verstellen **210** der dritten Handhabe 30 wird der Manipulatorzinken 98 in einem weiteren Schritt relativ zum/zu den Kopplungszinken 88 des Instrumentierabschnitts 18 in axialer Richtung translatorisch (nach distal) verstellt, und damit zwangsläufig der Cage 12 um die durch das Koppelglied 92 des Koppelzinkens 88 bzw. die Koppelglieder 98 der Kopplungszinken 88 verschwenkt, bis der Cage 12 im Bandscheibenfach 142 in seiner vorgegebenen Position angeordnet ist.

Ist der Cage 12 in sich gelenkig verstellbar ausgeführt, so kann der Anstellwinkel α des Kopfsegments 128 relativ zum Fußsegment 126 durch eine entsprechende Lordosierung der Wirbelsäule und eine damit einhergehendes Auslenkung des Kopf- und des Fußsegments relativ zueinander auf einen dazu korrespondierenden Wert eingestellt werden. Zum Arretieren **212** des Anstellwinkels α wird das Fixierelement 94 bzw. die Fixierschraube mittels der vierten Handhabe sowie der damit bewegungsgekoppelten ersten Arretierschiebers in den Einschraubbereich 138 des Cages 12 eingeschraubt.

In einem weiteren Schritt wird der erste Arretierschieber 34 aus seiner Arretierstellung in seine Freigabestellung in axialer Richtung translatorisch zurückbewegt. Der zweite Arretierschieber 38 wird durch Betätigen der am Instrumentiersegment 18 angeordneten Handhabe 40 in Richtung der Längsachse 22 des Positionierinstruments 14 aus seiner Arretierstellung in seine Freigabestellung translatorisch zurückbewegt. Der Instrumentierabschnitt 18 ist nun bezüglich seiner mechanischen Ankopplung am Cage entriegelt und kann in einem weiteren Schritt unter axialen Zug vom Cage 12 diskonnektiert und aus dem Operationssitus herausbewegt werden.

Zusammenfassend betrifft die Erfindung ein chirurgisches Cagepositioniersystem (10) für den implantologischen Ersatz einer Bandscheibe, bevorzugt im Bereich der lumbalen Wirbelsäule des Menschen mit einem Positionierinstrument (14) mit einem ersten Kopplungszinken (88) und mit einem ersten Manipulatorzinken (98), die jeweils mit einem Koppelglied (92) versehen sind. Der Manipulatorzinken (98) ist mittels einer Handhabe (26, 28, 30, 32, 40) relativ zum Kopplungszinken (88) in axialer Richtung verschiebbar.

Das Cagepositioniersystem umfasst einen Cage (12) mit einer Einstecköffnung (114) für das Instrumentiersegment (18) und mit Kopplungsmitteln (122, 124), über die die Koppelglieder (92) des Instrumentiersegments (18), jeweils drehgelenkig mit dem Cage (12) verkoppelbar sind, sodass der Cage (12) mittels des Manipulatorzinkens (98) um eine durch das Koppelglied (92) des Kopplungszinkens (88) und das Kopplungsmittel (122, 124) des Cages (12) definierte Drehachse D relativ zur Längsachse (22) des Positionierinstruments (14) verschwenkbar ist. Die Erfindung betrifft darüber hinaus einen Cage und ein Positionierinstrument.

## Patentansprüche

1. Chirurgisches Cagepositioniersystem (10) für den implantologischen Ersatz einer Bandscheibe, bevorzugt im Bereich der lumbalen Wirbelsäule des Menschen, umfassend:
- ein Positionierinstrument (14) mit einem Betätigungssegment (16) und mit einem Instrumentiersegment (18), das sich vom Betätigungssegment (16) in Richtung der Längsachse (22) des Positionierinstruments (14) wegerstreckt, wobei
das Instrumentiersegment (18) einen ersten Kopplungszinken (88) und einen ersten Manipulatorzinken (98) aufweist, die jeweils mit einem Koppelglied (92) versehen sind, wobei der Manipulatorzinken (98) mittels einer Handhabe (26, 28, 30, 32, 40) relativ zum Kopplungszinken (88) in axialer Richtung verschiebbar ist; und
- einen zu implantierenden Cage (12) mit einer Einstecköffnung (114) für das Instrumentiersegment (18) und mit Kopplungsmitteln (122, 124), über die die Koppelglieder (92) des Instrumentiersegments (18), jeweils drehgelenkig mit dem Cage (12) verkoppelbar sind,
wobei die Kopplungsmittel (122, 124) und die Koppelglieder (92) des am Cage angekoppelten Positionierinstruments (14) derart zusammenwirken, dass der Cage (12) mittels des Manipulatorzinkens (98) um eine durch das Koppelglied (92) des Kopplungszinkens (88) und das Kopplungsmittel (122, 124) des Cages (12) definierte erste Drehachse D relativ zur Längsachse (22) des Positionierinstruments (14) verschwenkbar ist,
**dadurch gekennzeichnet, dass**
durch das dem Koppelglied (92) des Manipulatorzinkens (98) zugeordnete Kopplungsmittel (122, 124) des Cages (12) beim Verschwenken des Cages (12) eine translatorische Verstellung des Koppelglieds (92) des Manipulatorzinkens (98) entlang einer nichtlinearen Bewegungsbahn (B) relativ zum Cage bewirkt ist, wobei das dem Koppelglied (92) des Manipulatorzinkens (98) zugeordnete Kopplungsmittel (122, 124) des Cages (12) als eine nichtlineare Nut oder als ein nichtlineares Langloch ausgeführt ist.

2. Cagepositioniersystem (10) nach Anspruch 1, **dadurch gekennzeichnet, dass** der Cage (12) durch Betätigen des Manipulationszinkens (98) über einen Schwenkwinkel β mit 0° ≤ β ≤ 80° relativ zur Längsachse (22) des Positionierwerkzeugs (14) verschwenkbar ist.

3. Cagepositioniersystem (10) nach einem der vorhergehenden Ansprüche, **dadurch gekennzeichnet, dass** das Positionierinstrument (14) einen ersten Arretierschieber (34) aufweist, der im oder am Grundkörper (24) des Instrumentiersegments (18) längsverschieblich gelagert ist und welcher relativ zum Kopplungszinken (88) in axialer Richtung zwischen einer Freigabestellung, in der der mit dem Cage (12) verkoppelte Kopplungszinken (88) vom Cage (12) diskonnektierbar ist, und einer Arretierstellung verschiebbar ist, in der der mit dem Cage (12) verkoppelte Kopplungszinken am Cage (12) arretiert ist, wobei der erste Arretierschieber (34) bevorzugt mittels einer am Instrumentiersegment (18) oder am Betätigungssegment (16) angeordneten Handhabe (26, 28, 30, 32, 40) betätigbar ist.

4. Cagepositioniersystem (10) nach einem der vorhergehenden Ansprüche, **dadurch gekennzeichnet, dass** das Instrumentiersegment (18) einen zweiten Kopplungszinken (88) aufweist, der zum ersten Kopplungszinken (88) bevorzugt parallel verlaufend angeordnet ist, wobei der erste Arretierschieber (34) in seiner Arretierstellung vorzugsweise zumindest abschnittsweise zwischen den beiden Kopplungszinken (88) angeordnet ist und ein Approximieren der beiden Kopplungszinken (88) sperrt.

5. Cagepositioniersystem (10) nach einem der vorhergehenden Ansprüche, **dadurch gekennzeichnet, dass** das Positionierinstrument (14) einen zweiten Arretierschieber (38) aufweist, der im oder am Grundkörper (24) des Instrumentiersegments (18) längsverschieblich gelagert ist und welcher relativ zum Manipulatorzinken (98) in axialer Richtung zwischen einer Freigabestellung, in der der mit dem Cage (12) verkoppelte Manipulatorzinkens (98) vom Cage diskonnektierbar ist, und einer Arretierstellung verschiebbar ist, in der der mit dem Cage (12) verkoppelte Manipulatorzinken (98) am Cage (12) arretiert ist.

6. Cagepositioniersystem (10) nach einem der vorhergehenden Ansprüche, **dadurch gekennzeichnet, dass** das Instrumentiersegment (18) einen zweiten Manipulatorzinken (98) aufweist, wobei der zweite Manipulatorzinken (98) und der erste Manipulatorzinken (98) synchronisiert in axialer Richtung verschiebbar sind und wobei der zweite Arretierschieber (38) in seiner Arretierstellung vorzugsweise zumindest abschnittsweise zwischen den beiden Manipulatorzinken (98) angeordnet ist und ein Approximieren derselben sperrt.

7. Cagepositioniersystem (10) nach einem der vorhergehenden Ansprüche, **dadurch gekennzeichnet, dass** das Instrumentiersegment (18) und das Betätigungssegment (16) jeweils als separate Baueinheiten ausgeführt sind, die miteinander lösbar verkoppelt sind.

8. Cagepositioniersystem (10) nach einem der vorhergehenden Ansprüche, **dadurch gekennzeichnet, dass** das Instrumentiersegment (18) für den Einmalgebrauch ausgelegt ist und das Betätigungssegment (16) für den Mehrfachgebrauch konzipiert ist, wobei das Betätigungssegment (16) vorzugswiese reversibel in seine Einzelteile zerlegbar und mehrfach autoklavierbar ist.

9. Cagepositioniersystem (10) nach einem der vorhergehenden Ansprüche, **dadurch gekennzeichnet, dass** der Cage (12) ein Fußsegment (126) und ein Kopfsegment (128) aufweist, die relativ zueinander verschwenkbar angeordnet sind.

10. Cagepositioniersystem (10) nach Anspruch 9, **dadurch gekennzeichnet, dass** das Fußsegment (126) und das Kopfsegment (128) über zumindest eine Nut-Federverbindung (136) aneinander geführt und miteinander verbunden sind.

11. Cagepositioniersystem (10) nach Anspruch 9 oder 10, **dadurch gekennzeichnet, dass** der Cage (12) ein Fixierelement (94) aufweist, durch das das Fußsegment (126) und das Kopfsegment (128) in ihrem jeweiligen Anstellwinkel α relativ zueinander lagefixierbar sind, wobei das Fixierelement (94) bevorzugt als eine Fixierschraube ausgeführt ist, die in einen, vorzugsweise zumindest abschnittsweise geriffelten, Einschraubbereich (138) des Cages (12) einschraubbar ist.

12. Cagepositioniersystem (10) nach Anspruch 11, **dadurch gekennzeichnet, dass** das Fixierelement (94) mittels eines Arretierschiebers (34, 38) betätigbar ist und mit diesem lösbar, vorzugsweise über eine Sollbruchstelle (96) oder ein in das Fixierelement (94) eingreifendes Werkzeugprofil (101) des Arretierschiebers (34, 38), bevorzugt einen Innensechskantschlüssel, verbunden ist, wobei der Arretierschieber (34, 38) bevorzugt mit einer Handhabe des Betätigungsabschnitts (16) drehfest verkoppelbar oder verkoppelt ist.

13. Cagepositioniersystem (10) nach einem der vorhergehenden Ansprüche, **dadurch gekennzeichnet, dass** das Betätigungssegment (16) und das Instrumentiersegment (18) ineinander steckbar und mittels eines, bevorzugt wippenartigen, Verriegelungsmittels (46), gegeneinander verriegelbar sind.

14. Cagepositioniersystem (10) nach einem der vorhergehenden Ansprüche, **dadurch gekennzeichnet, dass** das Positionierinstrument (14), bevorzugt das Betätigungssegment (16), eine Handhabe (26, 28, 30, 32, 40) zum Betätigen des Manipulatorzinkens (98) aufweist, wobei das Betätigungssegment bevorzugt eine Anzeige aufweist, anhand derer der jeweilige Schwenkwinkel β des Cages (12) relativ zur Längsachse (22) des Positionierwerkzeugs (14) ablesbar ist.

15. Cagepositioniersystem (10) nach einem der vorhergehenden Ansprüche, **dadurch gekennzeichnet, dass** jeder Kopplungszinken (88) am Grundkörper (24) des Instrumentiersegments (18) angeformt ist.

16. Cage (12) zum implantologischen Ersatz einer Bandscheibe für ein Positioniersystem nach einem der vorhergehenden Ansprüche, wobei der Cage vorzugsweise ein Kopfsegment (128) und ein Fußsegment (126) aufweist, die um eine Schwenkachse (132) relativ zueinander verschwenkbar sind.

17. Positionierinstrument (14) für ein Cagepositioniersystem (10) nach einem der vorhergehenden Ansprüche 1 bis 15, mit einem Betätigungssegment (16) und mit einem Instrumentiersegment (18), das sich vom Betätigungssegment (16) in Richtung der Längsachse (22) des Positionierinstruments (14) wegerstreckt, wobei das Instrumentiersegment (18) einen ersten Kopplungszinken (88) und einen ersten Manipulatorzinken (98) aufweist, die jeweils mit einem Koppelglied (92) versehen sind, wobei der Manipulatorzinken (98) mittels einer Handhabe (26, 28, 30, 32, 40) relativ zum Kopplungszinken (88) in axialer Richtung verschiebbar ist und wobei dessen Instrumentiersegment (18) bevorzugt für einen Einmalgebrauch und dessen Betätigungssegment (16) bevorzugt für den Mehrfachgebrauch konzipiert ist.

## Claims

1. A surgical cage positioning system (10) for the implantological replacement of an intervertebral disc, preferably in the region of the human lumbar spine, comprising:
- a positioning instrument (14) having an actuating segment (16) and having an instrumentation segment (18) which extends away from the actuating segment (16) in the direction of the longitudinal axis (22) of the positioning instrument (14), wherein the instrumentation segment (18) has a first coupling prong (88) and a first manipulator prong (98), each of which is provided with a coupling element (92), wherein the manipulator prong (98) is displaceable in the axial direction relative to the coupling prong (88) by means of a handle (26, 28, 30, 32, 40); and
- a cage (12) to be implanted, having an insertion opening (114) for the instrumentation segment (18) and having coupling means (122, 124) via which the coupling elements (92) of the instrumentation segment (18) can each be coupled to the cage (12) in an articulated manner, wherein the coupling means (122, 124) and the coupling elements (92) of the positioning instrument (14) coupled to the cage interact in such a way that the cage (12) is pivotable relative to the longitudinal axis (22) of the positioning instrument (14) by means of the manipulator prong (98) about a first axis of rotation D defined by the coupling element (92) of the coupling prong (88) and the coupling means (122, 124) of the cage (12),
**characterized in that**
the coupling means (122, 124) of the cage (12) associated with the coupling element (92) of the manipulator prong (98) causes a translational movement of the coupling element (92) of the manipulator prong (98) along a non-linear movement path (B) relative to the cage when the cage (12) is pivoted, wherein the coupling means (122, 124) of the cage (12) associated with the coupling element (92) of the manipulator prong (98) is preferably designed as a non-linear groove or a non-linear elongated hole.

2. The cage positioning system (10) according to claim 1, **characterized in that** the cage (12) can be pivoted relative to the longitudinal axis (22) of the positioning tool (14) through a pivot angle ß, where 0° ≤ ß ≤ 80°, by actuation of the manipulation prong (98).

3. The cage positioning system (10) according to either of the preceding claims, **characterized in that** the positioning instrument (14) has a first locking slide (34) which is mounted in a longitudinally displaceable manner in or on the main body (24) of the instrumentation segment (18), and which can be displaced relative to the coupling prong (88) in the axial direction between a releasing position, in which the coupling prong (88) coupled to the cage (12) can be disconnected from the cage (12), and a locking position, in which the coupling prong coupled to the cage (12) is locked on the cage (12), wherein the first locking slide (34) can preferably be actuated by means of a handle (26, 28, 30, 32, 40) arranged on the instrumentation segment (18) or on the actuating segment (16).

4. The cage positioning system (10) according to any of the preceding claims, **characterized in that** the instrumentation segment (18) has a second coupling prong (88) which is preferably arranged so as to extend in parallel with the first coupling prong (88), wherein the first locking slide (34), in its locking position, is preferably arranged at least in portions between the two coupling prongs (88) and blocks the two coupling prongs (88) from approaching each other.

5. The cage positioning system (10) according to any of the preceding claims, **characterized in that** the positioning instrument (14) has a second locking slide (38) which is mounted in a longitudinally displaceable manner in or on the main body (24) of the instrumentation segment (18) and which can be displaced relative to the manipulator prong (98) in the axial direction between a releasing position, in which the manipulator prong (98) coupled to the cage (12) can be disconnected from the cage, and a locking position, in which the manipulator prong (98) coupled to the cage (12) is locked on the cage (12).

6. The cage positioning system (10) according to any of the preceding claims, **characterized in that** the instrumentation segment (18) has a second manipulator prong (98), wherein the second manipulator prong (98) and the first manipulator prong (98) are synchronously displaceable in the axial direction, and the second locking slide (38), in its locking position, is preferably arranged at least in portions between the two manipulator prongs (98) and blocks said manipulator prongs from approaching each other.

7. The cage positioning system (10) according to any of the preceding claims, **characterized in that** the instrumentation segment (18) and the actuating segment (16) are each designed as separate structural units which are releasably coupled to each other.

8. The cage positioning system (10) according to any of the preceding claims, **characterized in that** the instrumentation segment (18) is designed for single use and the actuating segment (16) is designed for multiple use, wherein the actuating segment (16) can preferably be reversibly disassembled into its individual parts and can be autoclaved multiple times.

9. The cage positioning system (10) according to any of the preceding claims, **characterized in that** the cage (12) has a foot segment (126) and a head segment (128) which are arranged so as to be pivotable relative to each other.

10. The cage positioning system (10) according to claim 9, **characterized in that** the foot segment (126) and the head segment (128) are guided on each other and connected to each other via at least one tongue and groove connection (136).

11. The cage positioning system (10) according to claim 9 or 10,
**characterized in that** the cage (12) has a fixing element (94) by means of which the foot segment (126) and the head segment (128) can be fixed in position relative to each other in their relevant work angle a, wherein the fixing element (94) is preferably designed as a fixing screw which can be screwed into a screw-in region (138) of the cage (12), which is preferably corrugated at least in portions.

12. The cage positioning system (10) according to claim 11,
**characterized in that** the fixing element (94) can be actuated by means of a locking slide (34, 38) and is releasably connected to same, preferably via a predetermined breaking point (96) or a tool profile (101) of the locking slide (34, 38) that engages in the fixing element (94), preferably an Allen key, wherein the locking slide (34, 38) is preferably coupled or can be coupled to a handle of the actuating portion (16) in a rotationally fixed manner.

13. The cage positioning system (10) according to any of the preceding claims, **characterized in that** the actuating segment (16) and the instrumentation segment (18) can be plugged into each other and locked against each other by means of a preferably rocker-like locking means (46).

14. The cage positioning system (10) according to any of the preceding claims, **characterized in that** the positioning instrument (14), preferably the actuating segment (16), has a handle (26, 28, 30, 32, 40) for actuating the manipulator prong (98), wherein the actuating segment preferably has a display by means of which the relevant pivot angle β of the cage (12) relative to the longitudinal axis (22) of the positioning tool (14) can be read.

15. The cage positioning system (10) according to any of the preceding claims, **characterized in that** each coupling prong (88) is integrally formed on the main body (24) of the instrumentation segment (18).

16. A cage (12) for the implantological replacement of an intervertebral disc for a positioning system according to any of the preceding claims, wherein the cage preferably has a head segment (128) and a foot segment (126) which can be pivoted relative to each other about a pivot axis (132).

17. A positioning instrument (14) for a cage positioning system (10) according to any of the preceding claims 1 to 15, comprising an actuating segment (16) and an instrumentation segment (18) which extends from the actuating segment (16) in the direction of the longitudinal axis (22) of the positioning instrument (14), wherein the instrumentation segment (18) has a first coupling prong (88) and a first manipulator prong (98), each of which is provided with a coupling element (92), wherein the manipulator prong (98) is displaceable in the axial direction relative to the coupling prong (88) by means of a handle (26, 28, 30, 32, 40), and wherein its instrumentation segment (18) is preferably designed for single use and its actuating segment (16) is preferably designed for multiple use.

## Revendications

1. Système (10) de positionnement de cage chirurgicale, destiné au remplacement implantologique d'un disque intervertébral, de préférence dans la région lombaire de la colonne vertébrale humaine, comprenant :
- un instrument de positionnement (14) pourvu d'un tronçon d'actionnement (16) et d'un tronçon d'instrumentation (18) qui s'étend à partir dudit tronçon d'actionnement (16) dans la direction de l'axe longitudinal (22) de l'instrument de positionnement (14),
ledit tronçon d'instrumentation (18) comportant un premier fourchon d'accouplement (88) et un premier fourchon de manipulation (98) munis d'un organe de couplage (92) respectif, ledit fourchon de manipulation (98) pouvant être déplacé dans la direction axiale par rapport audit fourchon d'accouplement (88), au moyen d'une poignée (26, 28, 30, 32, 40) ; et
- une cage (12) à implanter, dotée d'un orifice d'emboîtement (114) dédié au tronçon d'instrumentation (18), et de moyens d'accouplement (122, 124) par l'intermédiaire desquels les organes de couplage (92) du tronçon d'instrumentation (18) peuvent être respectivement rattachés à la cage (12) avec articulation tournante, sachant que lesdits moyens d'accouplement (122, 124), et lesdits organes de couplage (92) de l'instrument de positionnement (14) rattaché à la cage, coopèrent de façon telle que ladite cage (12) puisse pivoter par rapport à l'axe longitudinal (22) dudit instrument de positionnement (14), au moyen du fourchon de manipulation (98), autour d'un premier axe de rotation D défini par l'organe de couplage (92) du fourchon d'accouplement (88) et par le moyen d'accouplement (122, 124) de ladite cage (12),
**caractérisé par le fait que**,
lors du pivotement de la cage (12), un déplacement translatoire de l'organe de couplage (92) du fourchon de manipulation (98) est provoqué par rapport à ladite cage, le long d'une trajectoire de mouvement (B) non linéaire, par le moyen d'accouplement (122, 124) de ladite cage (12) affecté audit organe de couplage (92) du fourchon de manipulation (98), sachant que ledit moyen d'accouplement (122, 124) de la cage (12), affecté audit organe de couplage (92) dudit fourchon de manipulation (98), est réalisé sous la forme d'une rainure non rectiligne ou d'un trou oblong non rectiligne.

2. Système (10) de positionnement de cage, selon la revendication 1, **caractérisé par le fait que** la cage (12) peut pivoter par rapport à l'axe longitudinal (22) de l'outil de positionnement (14), par actionnement du fourchon de manipulation (98), suivant un angle de pivotement β obéissant à 0° ≤ β ≤ 80°.

3. Système (10) de positionnement de cage, selon l'une des revendications précédentes, **caractérisé par le fait que** l'instrument de positionnement (14) est nanti d'un premier coulisseau d'arrêt (34) qui est monté à coulissement longitudinal dans, ou sur le corps de base (24) du tronçon d'instrumentation (18) et qui peut être déplacé dans la direction axiale, par rapport au fourchon d'accouplement (88), entre une position de libération dans laquelle ledit fourchon d'accouplement (88), rattaché à la cage (12), peut être dissocié d'avec ladite cage (12), et une position d'arrêt dans laquelle ledit fourchon d'accouplement, rattaché à la cage (12), est arrêté sur ladite cage (12), ledit premier coulisseau d'arrêt (34) pouvant être manoeuvré, de préférence, au moyen d'une poignée (26, 28, 30, 32, 40) implantée sur le tronçon d'instrumentation (18) ou sur le tronçon d'actionnement (16).

4. Système (10) de positionnement de cage, selon l'une des revendications précédentes, **caractérisé par le fait que** le tronçon d'instrumentation (18) est doté d'un second fourchon d'accouplement (88) agencé pour s'étendre, de préférence, parallèlement au premier fourchon d'accouplement (88), sachant que le premier coulisseau d'arrêt (34) occupant sa position d'arrêt est interposé entre les deux fourchons d'accouplement (88), de préférence au moins par zones, et interdit un rapprochement desdits deux fourchons d'accouplement (88).

5. Système (10) de positionnement de cage, selon l'une des revendications précédentes, **caractérisé par le fait que** l'instrument de positionnement (14) est pourvu d'un second coulisseau d'arrêt (38) qui est monté à coulissement longitudinal dans, ou sur le corps de base (24) du tronçon d'instrumentation (18) et qui peut être déplacé dans la direction axiale, par rapport au fourchon de manipulation (98), entre une position de libération dans laquelle ledit fourchon de manipulation (98), rattaché à la cage (12), peut être dissocié d'avec ladite cage, et une position d'arrêt dans laquelle ledit fourchon de manipulation (98), rattaché à la cage (12), est arrêté sur ladite cage (12).

6. Système (10) de positionnement de cage, selon l'une des revendications précédentes, **caractérisé par le fait que** le tronçon d'instrumentation (18) est muni d'un second fourchon de manipulation (98), sachant que ledit second fourchon de manipulation (98), et le premier fourchon de manipulation (98), peuvent accomplir des déplacements synchronisés dans la direction axiale, et sachant que le second coulisseau d'arrêt (38) occupant sa position d'arrêt est interposé, de préférence au moins par zones, entre les deux fourchons de manipulation (98) et interdit un rapprochement de ces derniers.

7. Système (10) de positionnement de cage, selon l'une des revendications précédentes, **caractérisé par le fait que** le tronçon d'instrumentation (18) et le tronçon d'actionnement (16) sont réalisés, à chaque fois, sous la forme d'unités structurelles distinctes couplées l'une à l'autre de manière libérable.

8. Système (10) de positionnement de cage, selon l'une des revendications précédentes, **caractérisé par le fait que** le tronçon d'instrumentation (18) est agencé en vue de l'usage unique et le tronçon d'actionnement (16) est conçu pour l'usage multiple, lequel tronçon d'actionnement (16) peut être démantelé en ses pièces individuelles, préférentiellement de manière réversible, et peut être traité plusieurs fois par autoclave.

9. Système (10) de positionnement de cage, selon l'une des revendications précédentes, **caractérisé par le fait que** la cage (12) comporte un tronçon d'embase (126) et un tronçon supérieur (128) agencés avec faculté de pivotement relatif.

10. Système (10) de positionnement de cage, selon la revendication 9, **caractérisé par le fait que** le tronçon d'embase (126) et le tronçon supérieur (128) sont guidés l'un sur l'autre, et reliés l'un à l'autre, par l'intermédiaire d'au moins une liaison (136) à rainure et languette.

11. Système (10) de positionnement de cage, selon la revendication 9 ou 10, **caractérisé par le fait que** la cage (12) est équipée d'un élément (94) de blocage à demeure, par l'intermédiaire duquel le tronçon d'embase (126) et le tronçon supérieur (128) peuvent être bloqués en position l'un vis-à-vis de l'autre, suivant leur angle d'incidence α respectif, ledit élément (94) de blocage à demeure étant réalisé, de préférence, sous la forme d'une vis de blocage à demeure pouvant être vissée dans une région de vissage (138) de ladite cage (12) qui, de préférence au moins par zones, est pourvue de striures.

12. Système (10) de positionnement de cage, selon la revendication 11, **caractérisé par le fait que** l'élément (94) de blocage à demeure peut être manoeuvré au moyen d'un coulisseau d'arrêt (34, 38) auquel il est relié, de manière libérable, préférentiellement par l'intermédiaire d'une zone (96) de rupture par destination ou d'un profil d'outillage (101) dudit coulisseau d'arrêt (34, 38), de préférence une clé mâle à six pans pénétrant dans ledit élément (94) de blocage à demeure, sachant que, de préférence, ledit coulisseau d'arrêt (34, 38) peut être, ou est couplé à une poignée de la région d'actionnement (16), avec verrouillage rotatif.

13. Système (10) de positionnement de cage, selon l'une des revendications précédentes, **caractérisé par le fait que** le tronçon d'actionnement (16) et le tronçon d'instrumentation (18) peuvent être emboîtés l'un dans l'autre et peuvent être verrouillés mutuellement au moyen d'un élément de verrouillage (46), préférentiellement du type pièce basculante.

14. Système (10) de positionnement de cage, selon l'une des revendications précédentes, **caractérisé par le fait que** l'instrument de positionnement (14), de préférence le tronçon d'actionnement (16), présente une poignée (26, 28, 30, 32, 40) dévolue à la manoeuvre du fourchon de manipulation (98), ledit tronçon d'actionnement étant préférentiellement pourvu d'un affichage à l'appui duquel il est possible de lire l'angle respectif β de pivotement de la cage (12) par rapport à l'axe longitudinal (22) de l'outil de positionnement (14).

15. Système (10) de positionnement de cage, selon l'une des revendications précédentes, **caractérisé par le fait que** chaque fourchon d'accouplement (88) est formé solidairement sur le corps de base (24) du tronçon d'instrumentation (18).

16. Cage (12) affectée au remplacement implantologique d'un disque intervertébral, dévolue à un système de positionnement conforme à l'une des revendications précédentes, ladite cage comprenant, de préférence, un tronçon supérieur (128) et un tronçon d'embase (126) aptes à pivoter l'un par rapport à l'autre autour d'un axe de pivotement (132).

17. Instrument de positionnement (14) destiné à un système (10) de positionnement de cage conforme à l'une des revendications 1 à 15, comprenant un tronçon d'actionnement (16) et un tronçon d'instrumentation (18) qui s'étend dans la direction de l'axe longitudinal (22) dudit instrument de positionnement (14), à partir dudit tronçon d'actionnement (16), lequel tronçon d'instrumentation (18) est muni d'un premier fourchon d'accouplement (88) et d'un premier fourchon de manipulation (98) dotés d'un organe de couplage (92) respectif, ledit fourchon de manipulation (98) pouvant être déplacé dans la direction axiale par rapport audit fourchon d'accouplement (88), au moyen d'une poignée (26, 28, 30, 32, 40), le tronçon d'instrumentation (18) dudit instrument étant conçu, de préférence, pour un usage unique et son tronçon d'actionnement (16) étant conçu, de préférence, pour l'usage multiple.
